# EUROPEAN PATENT APPLICATION

(11) **EP 4 671 385 A2**
(43) Date of publication of application: **31.12.2025**
(21) Application number: 25200037.7
(22) Date of filing: 07.08.2019
(51) Int. Cl.: C12Q 1/6886

(54) **TREATMENT OF SQUAMOUS CELL CARCINOMA**

(30) Priority: 07.08.2018 US 201862715634 P; 13.12.2018 EP 18212447
(62) Divisional of application: 19748564.2
(71) Applicant: Torqur AG, 4052 Basel (CH)
(72) Inventor: FABBRO, Doriano, 4052 Basel (CH); FREDERICK, Mitchell, Houston, TX 77005 (US); JOHNSON, Faye M., The Woodlands, TX 77380 (US); ROLLI, Melanie, 4052 Basel (CH); SCHMITZ-ROHMER, Debora, 4052 Basel (CH)
(74) Representative: Latscha Schöllhorn Partner AG

(57) **Abstract**

The present invention relates to a method of predicting the vulnerability of a squamous cell carcinoma (SCC) to inhibition by a PI3K inhibitor, preferably by a PI3K/mTOR inhibitor, including the selection of the patient predicted to benefit from therapeutic administration with the PI3K inhibitor, preferably of the PI3K/mTOR inhibitor. Moreover, the present invention relates to a method of treating a squamous cell carcinoma (SCC) of a mammal, preferably a human patient, comprising administering a therapeutically effective amount of a PI3K inhibitor, preferably a therapeutically effective amount of a PI3K/mTOR inhibitor to said mammal, preferably said human patient. Furthermore, the present invention relates to pharmaceutical compositions and kits associated with the inventive methods.

## Description

The present invention relates to a method of predicting the vulnerability of a squamous cell carcinoma (SCC) to inhibition by a PI3K inhibitor, preferably by a PI3K/mTOR inhibitor, including the selection of the patient predicted to benefit from therapeutic administration with the PI3K inhibitor, preferably of the PI3K/mTOR inhibitor. Moreover, the present invention relates to a method of treating a squamous cell carcinoma (SCC) of a mammal, preferably a human patient, comprising administering a therapeutically effective amount of a PI3K inhibitor, preferably a therapeutically effective amount of a PI3K/mTOR inhibitor to said mammal, preferably said human patient. Furthermore, the present invention relates to pharmaceutical compositions and kits associated with the inventive methods.

### RELATED ART

More than 90% of tumors in the head and neck are squamous cell carcinomas (HNSCC) (Ferlay, Soerjomataram et al. 2015, The Cancer Genome Atlas 2015). Head and neck squamous cell carcinoma (HNSCC) is a lethal, disabling, disfiguring cancer and the seventh leading cause of cancer-related deaths globally with more than 375,000 individuals dying from this cancer yearly (Ferlay, Soerjomataram et al. 2015). In this disease, treatment morbidity is high and recurrence is common. Although immunotherapy has had a striking effect in some patients with metastatic or recurrent HNSCC, the majority of patients still progress. Standard chemotherapy (methotrexate, docetaxel, others) or cetuximab beyond first line therapy benefits less than 15% of patients. Except for Cetuximab, for which there is no biomarker to predict response, there are no molecular targeted therapies approved for treating HNSCC patients, identifying a significant translational knowledge gap. Although the unbiased genomic characterization of multiple cancers has fundamentally changed our approach to cancer therapy and translational research, this revolution has not yet affected therapy for HNSCC and targeted therapy based on biomarkers does not yet exist for HNSCC.

Recent genomic characterization of HNSCC by several independent groups has demonstrated remarkable complexity but also four common driver-signaling pathways (Agrawal, Frederick et al. 2011, Stransky, Egloff et al. 2011, Iglesias-Bartolome, Martin et al. 2013, Pickering, Zhang et al. 2013). Of the mitogenic pathways affected, the PI3K/AKT/mTOR pathway is the most often altered in HNSCC, with ~80% of HNSCC tumors containing molecular alterations in one or more components of the pathway (Iglesias-Bartolome, Martin et al. 2013, Lui, Hedberg et al. 2013). In HNSCC, the PI3K/mTOR pathway is altered in 54% of patients including copy number alterations in PIK3CA (35%), PTEN (6%), RICTOR (7%), AKT1 (3%), PIK3R1 (2%), and MTOR (3%) (Cerami, Gao et al. 2012). In particular PIK3CA is the third most frequently altered gene (18%) in HNSCC with frequent hotspot mutations in the helical (E542K or E545K) and kinase (H1047R) domains in human papilloma virus (HPV)-negative HNSCC patients and mutations in the helical domain in HPV-positive HNSCC patients (Iglesias-Bartolome, Martin et al. 2013). Clinical responses to PI3K/AKT/mTOR pathway inhibitors have been modest and short-lived in most solid tumors (Rodon, Dienstmann et al. 2013, Fruman and Rommel 2014) and there are no biomarkers to guide patient selection (Fruman and Rommel 2014). Use of PIK3CA mutation as a biomarker is inconclusive, with studies showing both increased sensitivity (Di Nicolantonio, Arena et al. 2010, Lui, Hedberg et al. 2013) and no differential response to PI3K/AKT/mTOR inhibitors in clinical trials (Janku, Tsimberidou et al. 2011, Janku, Wheler et al. 2012, Jimeno, Bauman et al. 2015). Consistent with the clinical findings, HNSCC cell lines and patient derived xenografts (PDXs) with PIK3CA mutations were more sensitive to PI3K/mTOR pathway inhibitors than PIK3CAWT HNSCC cells but these drugs led to only cell-cycle arrest with no apoptosis in the mutant cell lines (Lui, Hedberg et al. 2013, Mazumdar, Byers et al. 2014, Jimeno, Bauman et al. 2015). The frequent activation of the PI3K/AKT/mTOR pathway in HNSCC, the availability of pharmacologic inhibitors and the pathway's importance in cancer cell signaling make this pathway a promising target for needed improved systemic therapy and to identify potential targetable alterations within this pathway (Mazumdar, Byers et al. 2014).

NOTCH1 can function in cancer as either a tumor suppressor or oncogene depending upon the tissue specific context (Mao 2015, Nowell and Radtke 2017). NOTCH family receptors regulate cell fate decisions, lineage commitment, and differentiation (Mao 2015, Nowell and Radtke 2017). Humans have four NOTCH family receptors (NOTCH1-4) which are activated in a juxtacrine manner by any of five canonical ligands (Jagged-1, -2, Delta-like ligand 1, -3, and -4) expressed on neighboring cells (D'Souza, Miyamoto et al. 2008, Agrawal, Frederick et al. 2011). Aberrant NOTCH signaling has been implicated in the development, progression, and prognosis of many cancer types. However, actual genomic alterations to NOTCH family receptor genes mainly occur in NOTCH1, and are found frequently in only some tumor types, including T-cell acute leukemia (T-ALL) (Ferrando 2009), adenoid cystic carcinoma (Ho, Kannan et al. 2013, Ferrarotto, Mitani et al. 2017), and squamous cell carcinomas of the head and neck (Pickering, Zhang et al. 2013), skin (Pickering, Zhou et al. 2014), esophagus (Agrawal, Jiao et al. 2012), and lung (The Cancer Genome Atlas 2015). Importantly, the majority of *NOTCH1* mutations in T-ALL and adenoid cystic carcinoma lead to an over-activation of the protein (Ferrando 2009; Ho, Kannan et al. 2013; Ferrarotto, Mitani et al. 2017). The oncogenic function of over-activated *NOTCH1* in T-ALL has been extensively studied (Hales et al. 2014). Moreover, Asian populations with HNSCC have shown activating *NOTCH1* mutations (Fukusumi et al 2018). NOTCH1 receptors are expressed after cleavage of a larger NOTCH1 precursor protein into extracellular and intracellular peptides that heterodimerize at the cell surface through specific heterodimerization domains (HDs) (Nowell and Radtke 2017). Ligand binding to the extracellular EGF-like repeats on NOTCH1 receptors creates mechanical tension exposing the molecule to stepwise cleavage at the S2 site by α-secretases and finally at the S3 cleavage site by γ-secretase to release intracellular cleaved NOTCH1 (cl-NOTCH1; NOTCH1 intracellular domain = NCID1). cl-NOTCH1 translocates to the nucleus and binds other transcription co-factors, altering expression of genes (Nowell and Radtke 2017) .

NOTCH1 is among the top five most frequently mutated genes in HNSCC (Stransky, Egloff et al. 2011, The Cancer Genome Atlas 2015) and NOTCH1 mutations occur at high frequency of about 20% in untreated and recurrent HNSCC (Cancer Genome Atlas Research, 2013; Morris et al., 2017). Recently, it has been described that NOTCH1 inactivating mutation mediates sensitivity to PI3K/mTOR inhibition in HNSCC and that HNSCC cell lines harboring NOTCH1 mutation underwent apoptosis after PI3K/mTOR pathway inhibition *in vitro* and decreased tumor size *in vivo* (Johnson, Sambandam et al. 2016, Sambandam, Shen et al. 2017) (Sambandam et al. 2018).

Several PI3K/mTOR inhibitors are known and have been described (Courtney, Corcoran et al. 2010, Maira 2011, Rodon, Dienstmann et al. 2013, Fruman and Rommel 2014, Beaufils, Cmiljanovic et al. 2017, Bohnacker, Prota et al. 2017, Fruman, Chiu et al.). Among them bimiralisib is a pan-class I PI3K/mTOR antagonist that potently inhibits PI3Kα and mTOR (IC50 = 2 to 25 nM), with less potency against PI3Kβ (IC50 = 820 nM) (Beaufils, Cmiljanovic et al. 2017, Bohnacker, Prota et al. 2017). It is highly selective and does not significantly inhibit other protein kinases tested in biochemical assays (KINOMEscan), or receptors or ion channels in the CEREP Bioprint profile (Beaufils, Cmiljanovic et al. 2017, Bohnacker, Prota et al. 2017) bimiralisib is administered orally and crosses the blood-brain barrier (Beaufils, Cmiljanovic et al. 2017, Bohnacker, Prota et al. 2017). Human pharmacokinetic data demonstrate rapid drug absorption (Tₘₐₓ <2 h), terminal half-life of 51 hours, and Cₘₐₓ of 0.96 to 1.46 µg/mL (2.3 to 3.5 µM) (Wicki, Brown et al. 2018). Pharmacodynamic data demonstrate marked decreases of pAKT, pS6 and p4EBP in tumor tissue at therapeutic doses (Beaufils, Cmiljanovic et al. 2017, Bohnacker, Prota et al. 2017, Wicki, Brown et al. 2018).

Taken all of the above into consideration, there is an urgent need for developing a biomarker-based targeted therapy for the treatment of SCCs, in particular for HNSCC, which enables selection and treatment of patients with SCCs, in particular HNSCC, which are likely to benefit from said therapy, thus creating a positive risk/benefit ratio for patients.

### SUMMARY OF THE INVENTION

The present invention provides a method of predicting the vulnerability of squamous cell carcinoma (SCC) to inhibition by a PI3K inhibitor, preferably by a PI3K/mTOR inhibitor, in particular by the very preferred PI3K/mTOR inhibitor named bimiralisib, preferably based on a specific selection of biomarkers in association with *NOTCH1* mutations harbored in said SCCs, which mutations are considered to lead to loss of function (LoF). Such specific selection of *NOTCH1* mutations harbored in said SCCs are considered to lead to loss of function (LoF) while excluding *NOTCH1* mutations believed to lead to an over-activation of the protein or acting in an oncogenic manner. Thus, the specific selection of *NOTCH1* mutations in accordance with the present invention increases the treatment success. The present invention thus provides a novel targeted therapy for treating SCC of a mammal, preferably of a human patient, in particular for treating head and neck SCC (HNSCC) human patients that have been selected as to benefit from said targeted therapy. In detail, the present invention preferably relates to targeting SCC harboring specific *NOTCH1* mutations considered to be loss of function (LoF) while excluding SCC harboring *NOTCH1* mutations believed to lead to an over-activation and thus to an oncogenic effect. In particular, the present invention relates to targeting HNSCC harboring said specific *NOTCH1* LoF mutations. To our knowledge, the present invention comprises the first approach of targeting the loss of a tumor suppressor in human patients.

Importantly, the effective treatment with bimiralisib of a human patient with heavily pretreated metastatic HNSCC with a SCC of the tongue harboring a *NOTCH1* LoF mutation in accordance with the present invention was conducted in the context of a clinical trial (Example 4). After 6 weeks of treatment with bimiralisib, target lesions (metastases) of the patient had regressed remarkably (by more than 80%). The patient remained on bimiralisib treatment for several months until she passed away due to an event unrelated to bimiralisib. Moreover, first results from a subsequent clinical study showed that another patient with pretreated metastatic HNSCC harboring a *NOTCH1* mutation in accordance with the present invention and with lung metastases was beneficially treated with the very preferred PI3K/mTOR inhibitor bimiralisib in the described study (Example 5). The patient has been treated with bimiralisib for over five months and continues to benefit from treatment. Bimiralisib has completely stopped the growth of his metastases as evidenced by radiological tumor assessments. Thus, in very preferred examples and studies of the present invention, the efficacy of the very preferred PI3K inhibitor and PI3K/mTOR inhibitor, respectively, bimiralisib, in patients whose HNSCC harbored a *NOTCH1* LoF mutation in accordance with the present invention was demonstrated. It was thus made plausible and is expected that the treatment of said HNSCC patients with PI3K inhibitor and PI3K/mTOR inhibitor, respectively, and in particular, with bimiralisib will result in the vulnerability of the HNSCC leading to its rapid shrinkage presumably due to apoptosis.

The prediction of the vulnerability of SCC, in particular HNSCC, and the selection criteria of the mammal, preferably human patients predicted to benefit from the targeted therapy in accordance with the present invention is, in particular, based on the occurrence, nature and distribution pattern of *NOTCH1* mutations and the inventive specific selection of *NOTCH1* mutations considered to be loss of function (LoF) mutations in accordance with the present invention. In particular, the present invention identifies specific selection criteria for said *NOTCH1* mutations to predict the vulnerability of SCC, in particular HNSCC, and to predict the increased efficacy of a PI3K inhibitor and PI3K/mTOR inhibitor, respectively, and in particular of bimiralisib, for the treatment of SCC, in particular HNSCC.

In summary, the present invention establishes a biomarker-based targeted therapy for the treatment of SCCs and in particular for HNSCC that facilitate selection and treatment of patients with SCCs and in particular HNSCC which are likely to benefit from the inventive treatment, in particular from the preferred treatment with bimiralisib. The present invention, thus, advantageously and preferably, creates a favorable risk-benefit ratio for the mammal, preferably human patients by limiting said inventive treatment, preferably said bimiralisib treatment to said patients that will likely benefit from bimiralisib using the principles of personalized medicine.

Thus, in a first aspect, the present invention provides a method of predicting the vulnerability of a squamous cell carcinoma (SCC) to inhibition by a PI3K inhibitor, preferably by a PI3K/mTOR inhibitor, wherein said method comprises
(a) identifying the status of a biomarker from tumor material from a mammal, preferably from a human patient, wherein the biomarker is selected from the group consisting of
   (i) sequenced tumor DNA, preferably human tumor DNA, to identify one or more mutations in the *NOTCH1 gene,* preferably in the human *NOTCH1* gene of SEQ ID NO:1, encoding the NOTCH1 protein, preferably the human NOTCH1 protein of SEQ ID NO:2;
   (ii) protein level of cleaved NOTCH1 intracellular domain, preferably the protein level of human cleaved NOTCH1 intracellular domain (NICD1; SEQ ID NO:3); and
   (iii) a combination of biomarker (i) and (ii);
(b) comparing the status of the biomarker in said tumor material to a normal status of the biomarker; and
(c) selecting the mammal, preferably the human patient, as being predicted to benefit from therapeutic administration of the PI3K inhibitor, preferably of the PI3K/mTOR inhibitor, if
   (i) said mammal's, preferably human patient's, SCC harbors one or more *NOTCH1* mutations, wherein said *NOTCH1* mutation is not
      a. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2;
      b. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; or
      c. a mutation in the splice donor boundary (Exon 33), or in the acceptor boundary (Exon 34) of said *NOTCH1* gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1; or
   (ii) said mammal's, preferably human patient's, SCC comprises cleaved NOTCH1 intracellular domain protein, preferably human cleaved NOTCH1 intracellular domain (NICD1; SEQ ID NO:3) in an amount incompatible with NOTCH1 loss-of-function; or
   (iii) a combination of (i) and (ii).

The Cancer Genome Atlas (TCGA) describes nearly 100 *NOTCH1* mutations identified for T-ALL and HNSCC with the pattern, site of occurrence and nature of said *NOTCH1* mutations (Cancer Genome Atlas 2015, Nowell and Radtke 2017). The comparison of the pattern of said *NOTCH1* mutations led us to conclude the oncogenic *NOTCH1* mutations found in T-ALL occur in a hotspot of mostly missense mutations within the negative regulatory HD domain or in a second hotspot of mostly truncating mutations near the C-terminus, deleting the PEST domain and causing increased stabilization of activated *NOTCH1* in the nucleus (Ferrando 2009, Nowell and Radtke 2017). However, in HNSCC the truncating mutations are scattered throughout the protein but not in the PEST domain, and the missense mutations cluster in the extracellular EGF-ligand binding domains. Unlike T-ALL, only 1% of *NOTCH1* missense mutations in HNSCC are in either the negative regulatory or PEST domains. Without being bound, it is believed that mutations in the splice donor boundary (Exon 33), or in the acceptor boundary (Exon 34) of said *NOTCH1* gene are able to truncate said *NOTCH1* gene, in particular, said human *NOTCH1* gene of SEQ ID NO:1, within said TAD domain or said PEST domain.

In accordance with the present invention, SCCs considered to harbor a LoF mutation in the *NOTCH1* gene are more likely to respond to (i.e., to shrink due to believed increased apoptosis) a PI3K inhibitor, preferably to a PI3K/mTOR inhibitor, in particular to bimiralisib. Detection of one or more of said LoF mutations in the *NOTCH1* gene predicts that the patient will benefit from treatment with the PI3K inhibitor, preferably PI3K/mTOR inhibitor, and in particular with bimiralisib.

In a further aspect, the present invention provides a method of predicting the vulnerability of a tumor material from a squamous cell carcinoma (SCC) of a mammal, preferably of a human patient, to a PI3K inhibitor, preferably a PI3K/mTOR inhibitor, wherein said method comprises contacting a tumor material from a squamous cell carcinoma (SCC) with said PI3K inhibitor, preferably said PI3K/mTOR inhibitor, and
(a) identifying the status of a biomarker of said tumor material, wherein the biomarker is selected from the group consisting of
   (i) sequenced tumor DNA, preferably human tumor DNA, to identify one or more mutations in the *NOTCH1* gene, preferably in the human *NOTCH1* gene of SEQ ID NO:1 , encoding the NOTCH1 protein, preferably the human NOTCH1 protein of SEQ ID NO:2 ;
   (ii) protein level of cleaved NOTCH1 intracellular domain, preferably the protein level of human cleaved NOTCH1 intracellular domain (NICD1; SEQ ID NO:3); and
   (iii) a combination of biomarker (i) and (ii);
(b) comparing the status of the biomarker in said tumor material to a normal status of the biomarker; and
(c) determining the vulnerability of the tumor material to a PI3K inhibitor, preferably of a PI3K/mTOR inhibitor based on the difference of the status of the biomarker in the tumor material and the normal status, and wherein determining the tumor material of said mammal, preferably said human patient, as being vulnerable to a PI3K inhibitor, preferably of a PI3K/mTOR inhibitor, if
   (i) said mammal's, preferably human patient's, tumor material harbors one or more *NOTCH1* mutations, wherein said *NOTCH1* mutation is not
      a. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2;
      b. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; or
      c. a mutation in the splice donor boundary (Exon 33), or in the acceptor boundary (Exon 34) of said *NOTCH1* gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1; or
   (ii) said mammal's, preferably human patient's, tumor material comprises cleaved NOTCH1 intracellular domain protein, preferably human cleaved NOTCH1 intracellular domain (NICD1; SEQ ID NO:3) in an amount incompatible with NOTCH1 loss-of-function; or
   (iii) a combination of (i) and (ii).

In another aspect, the present invention provides a method of treating a squamous cell carcinoma (SCC) of a mammal, preferably a human patient, comprising administering a therapeutically effective amount of a PI3K inhibitor, preferably a therapeutically effective amount of a PI3K/mTOR inhibitor to said mammal, preferably said human patient, wherein
(i) said mammal's, preferably human patient's, SCC harbors one or more *NOTCH1* mutations, wherein said *NOTCH1* mutation is not
   a. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2;
   b. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; or
   c. a mutation in the splice donor boundary (Exon 33), or in the acceptor boundary (Exon 34) of said *NOTCH1* gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1; or
(ii) said mammal's, preferably human patient's, SCC comprises cleaved NOTCH1 intracellular domain protein, preferably human cleaved NOTCH1 intracellular domain (NICD1; SEQ ID NO:3) in an amount incompatible with NOTCH1 loss-of-function; or
(iii) a combination of (i) and (ii).

In another aspect, the present invention provides a method of treating a squamous cell carcinoma (SCC) of a mammal, preferably a human patient with a PI3K inhibitor, preferably a PI3K/mTOR inhibitor, wherein said method comprises
(a) selecting said mammal, preferably said human patient, as being predicted to benefit from said treatment with said PI3K inhibitor, preferably said PI3K/mTOR inhibitor, wherein said selecting comprises
   (i) identifying the status of a biomarker from tumor material from said mammal, preferably from said human patient, wherein the biomarker is selected from the group consisting of
      a. sequenced tumor DNA, preferably human tumor DNA, to identify one or more mutations in the *NOTCH1* gene, preferably in the human *NOTCH1* gene of SEQ ID NO:1, encoding the NOTCH1 protein, preferably the human NOTCH1 protein of SEQ ID NO:2;
      b. protein level of cleaved NOTCH1 intracellular domain, preferably the protein level of human cleaved NOTCH1 intracellular domain (NICD1; SEQ ID NO:3); and
      c. a combination of biomarker (i) and (ii);
   (ii) comparing the status of the biomarker in said tumor material to a normal status of the biomarker; and
   (iii) selecting the mammal, preferably the human patient, as being predicted to benefit from said treatment with said PI3K inhibitor, preferably said PI3K/mTOR inhibitor, if
      a. said mammal's, preferably human patient's, SCC harbors one or more *NOTCH1* mutations, wherein said *NOTCH1* mutation is not
         i. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2;
         ii. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2 (full length human NOTCH1 protein]
         iii. a mutation in the splice donor boundary (Exon 33), or in the acceptor boundary (Exon 34) of said *NOTCH1* gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1; or
      b. said mammal's, preferably human patient's, SCC comprises cleaved NOTCH1 intracellular domain protein, preferably human cleaved NOTCH1 intracellular domain (NICD1; SEQ ID NO:3) in an amount incompatible with NOTCH1 loss-of-function; or
      c. a combination of a. and b.;
(b) administering a therapeutically effective amount of said PI3K inhibitor, preferably a therapeutically effective amount of said PI3K/mTOR inhibitor to said selected mammal, preferably said selected human patient.

In another aspect, the present invention provides a kit for selecting a mammal, preferably a human patient, with squamous cell carcinoma being predicted to benefit or not to benefit from administration of a PI3K inhibitor, preferably of a PI3K/mTOR inhibitor, the kit comprising:
(a) a means for identifying in a tumor material a status of a biomarker selected from the group consisting of
   (i) sequenced tumor DNA, preferably human tumor DNA, to identify one or more mutations in the *NOTCH1* gene, preferably in the human *NOTCH1* gene of SEQ ID NO:1, encoding the NOTCH1 protein, preferably the human NOTCH1 protein of SEQ ID NO:2;
   (ii) protein level of cleaved NOTCH1 intracellular domain (cl-NOTCH1, [NICD1]; SEQ ID NO:3), wherein preferably said NICD1 is determined by immunohistochemistry (IHC); and
   (iii) a combination of biomarker (i) and (ii);
(b) a means for identifying the normal status.

In a further aspect, the present invention provides a pharmaceutical composition comprising a therapeutically effective amount of a PI3K inhibitor, preferably a therapeutically effective amount of a PI3K/mTOR inhibitor for use in treatment of a squamous cell carcinoma (SCC) of a mammal, preferably a human patient, wherein
(i) said mammal's, preferably human patient's, SCC harbors one or more *NOTCH1* mutations, wherein said *NOTCH1* mutation is not
   a. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2;
   b. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; or
   c. a mutation in the splice donor boundary (Exon 33), or in the acceptor boundary (Exon 34) of said *NOTCH1* gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1; or
(ii) said mammal's, preferably human patient's, SCC comprises cleaved NOTCH1 intracellular domain protein (cl-NOTCH1, [NICD1]; SEQ ID NO:3) in an amount incompatible with NOTCH1 loss-of-function; or
(iii) a combination of (i) and (ii).

In another aspect, the present invention pharmaceutical composition comprising a therapeutically effective amount of a PI3K inhibitor, preferably a therapeutically effective amount of a PI3K/mTOR inhibitor for use in treatment of a squamous cell carcinoma (SCC) of a mammal, preferably a human patient, wherein said mammal, preferably said human patient is selected to benefit from said treatment with said PI3K inhibitor, preferably said PI3K/mTOR inhibitor, and wherein said selecting comprises
(i) identifying the status of a biomarker from tumor material from said mammal, preferably from said human patient, wherein the biomarker is selected from the group consisting of
   a. sequenced tumor DNA, preferably sequenced human tumor DNA, to identify one or more mutations in the NOTCH1 gene, preferably in the human *NOTCH1* gene of SEQ ID NO:1, encoding the NOTCH1 protein, preferably the human NOTCH1 protein of SEQ ID NO:2;
   b. protein level of cleaved NOTCH1 intracellular domain (cl-NOTCH1, [NICD1]; SEQ ID NO:3), wherein preferably said NICD1 is determined by immunohistochemistry (IHC); and
   c. a combination of biomarker (i) and (ii);
(ii) comparing the status of the biomarker in said tumor material to a normal status of the biomarker; and
(iii) selecting the mammal, preferably the human patient, as being predicted to benefit from said treatment with said PI3K inhibitor, preferably said PI3K/mTOR inhibitor, if
   a. said mammal's, preferably human patient's, SCC harbors one or more *NOTCH1* mutations, wherein said *NOTCH1* mutation is not
      i. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2;
      ii. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2 (full length human NOTCH1 protein]
      iii. a mutation in the splice donor boundary (Exon 33), or in the acceptor boundary (Exon 34) of said NOTCH1 gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1; or
   b. said mammal's, preferably human patient's, SCC comprises cleaved NOTCH1 intracellular domain protein (cl-NOTCH1, [NICD1]; SEQ ID NO:3) in an amount incompatible with NOTCH1 loss-of-function; or
   c. a combination of a. and b..

Further aspects and embodiments of the present invention will be become apparent as this description continues.

### DESCRIPTION OF FIGURES

- FIG. 1:: Bimiralisib inhibits PI3K pathway signaling in HNSCC. Four HNSCC cell lines were treated with the indicated concentrations of bimiralisib for 2 hours. Cells were lysed and then resolved by SDS PAGE. Western blot analysis was performed using the indicated antibodies.
- FIG. 2A:: Genotype of HNSCC cell lines. Whole exome sequencing (WES) was performed on 66 established HNSCC lines. A number of these cell lines were mutant for *NOTCH1* or had PIK3CA mutations in known hotspots, but they also had additional driver mutations frequently observed in HNSCC.
- FIG. 2B:: Genotype of HNSCC cell lines. Non-canonical mutations or possibly SNPS were excluded from the analysis. a: non-canonical mutation excluded from analysis; b: possible SNP excluded from *NOTCH1* analysis; *non characterized *NOTCH1* mutation.
- FIG. 3A:: Loss and restoration of *NOTCH1* expression. Total *NOTCH1* is absent from cells with truncating mutations (ns =non sense mutation, fs = frames shift mutation), but present in wt cells or in HN31 with a missense (ms) mutation.
- FIG. 3B:: Loss and restoration of *NOTCH1* expression. Infection with full length wt *NOTCH1* (NFL1) but not empty vector (MigR1) restores *NOTCH1* activation detected as cleaved-NOTCH1. Jag1= NOTCH ligand. FC = protein; UN = untreated.

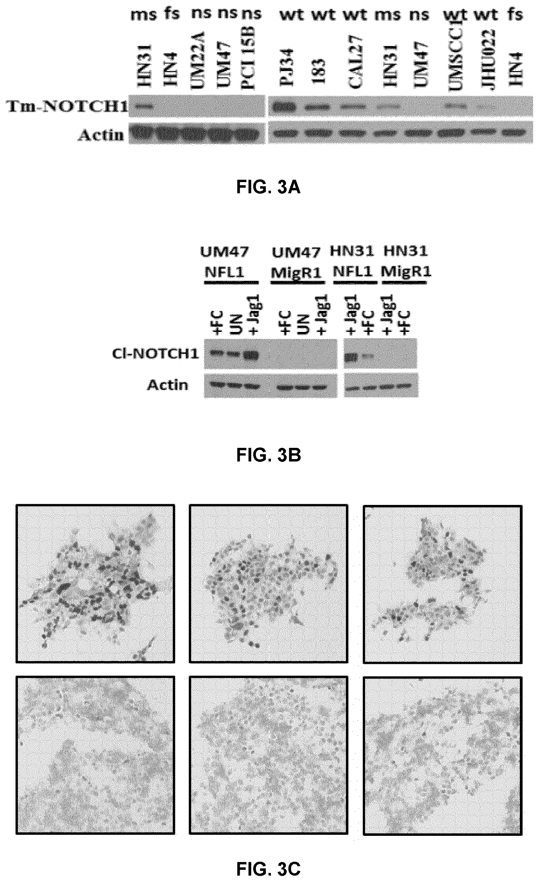

- FIG. 3C:: Loss and restoration of *NOTCH1* expression. The presence and residual levels of activated *NOTCH1* is assessed by determination of the expression of cleaved NOTCH1 (cl-NOTCH1) in tumors or tumor cells, typically and preferably, by immunohistochemistry (IHC) according to published methods (Kluk, Ashworth et al. 2013, Rettig, Chung et al. 2015), with an antibody that specifically detects cl-NOTCH1. Upper panel shows section of paraffin-embedded pellets of FADU HNSCC cell line expressing NOTCH1wt. Lower panel shows section of paraffin-embedded pellets of FADU HNSCC cell line where the NOTCH1wt was deleted by CRISPR/Cas9.
- FIG. 4A:: HNSCC cells with *NOTCH1*-LoF mutants are more sensitive to bimiralisib than *NOTCH1*wt cells *in vitro.* Bimiralisib at 5 µM induces death in HNSCC cell lines with *NOTCH1* mutations. Cell death as measured with BrdU TUNEL was significantly increased in HNSCC cell lines with *NOTCH1* mutations (dotted) but not in HNSCC lines with wt *NOTCH1* (grey) or *PIK3CA* mutations (black) 48 hours after treatment with bimiralisib. Apoptotic death in *NOTCH1* mutants, but not wt cell lines, was confirmed with cleaved PARP and Caspase 3.

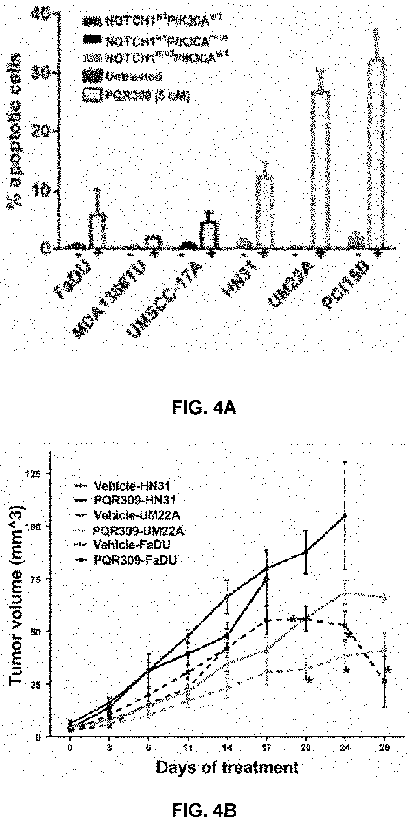

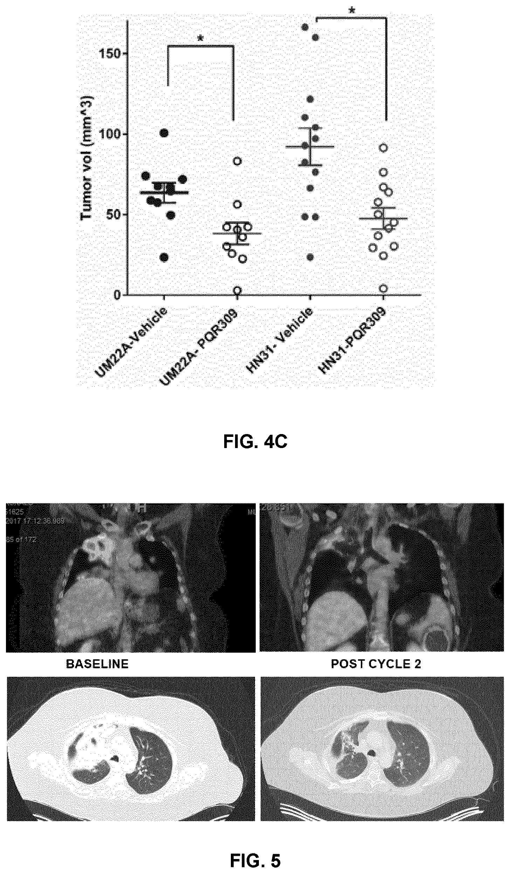

- FIG. 4B:: HNSCC tumors harboring *NOTCH1*-LoF mutants are more sensitive to bimiralisib than *NOTCH1*wt cells *in vivo.* Bimiralisib (50 mg/kg QD, PO) treatment in *NOTCH1*^{mut} and WT orthotopic xenografts *in vivo.* Reduction in tumor growth was seen in mutant lines compared to FaDU. In detail, *in vivo* response of bimiralisib (50 mg/kg) PO once daily against 2 orthotopic tongue xenograft model with 2 NOTCH1mutant lines. After the growth of the xenografts, the mice were randomized and treated with bimiralisib daily for 28 days. The figure shows tumor growth curve over time. The solid lines are tumor volume of UM22A and HN31 (*NOTCH1* inactivating mutants) when treated with vehicle or when FaDu tumors with NOTCH1wt were treated once daily with 50mg/kg bimiralisib PO. Dotted lines are the tumor volumes of UM22A and HN31 after PO once daily treatment with 50 mg/kg bimiralisib.
- FIG. 4C:: HNSCC tumors harboring *NOTCH1* -LoF mutants are more sensitive to bimiralisib than *NOTCH1*wt cells *in vivo.* There was significant reduction of tumor growth (p<0.05) after treatment once daily with bimiralisib (50 mg/kg) in the UM22A and HN31 *NOTCH1* inactivating mutant tumors.
- FIG. 5:: Treatment of a patient with metastatic HNSCC harboring a *NOTCH1* -LoF mutation in accordance with the present invention. Clinical response with bimiralisib treatment of a human patient with heavily pretreated metastatic HNSCC with a SCC of the tongue harboring a *NOTCH1* LoF mutation (L250fs*6) with a lung metastasis. After 6 weeks of treatment with bimiralisib said lung metastasis regressed as determined by PET/CT (upper panel longitudinal scan and lower panel cross section). The patient remained on the study for 8 months. The maximal response was an 89% reduction in tumor size.
- FIG. 6:: Flow-diagram for selecting *NOTCH1* -LoF mutations in accordance with a preferred embodiment of the present invention. Screening patients for eligibility in study trial of Example 5 for bimiralisib. Patients with *NOTCH1* mutations in regions associated with activation including TAD/PEST domains or mutations in LNR and HD domains that are not truncating are excluded. Splice mutations in Exon 33 or 34 are also excluded, but patients with *NOTCH1* mutation in all other regions are eligible.

### DETAILED DESCRIPTION OF THE INVENTION

Unless defined otherwise, all technical and scientific terms used herein have the same meanings as commonly understood by one of ordinary skill in the art to which this invention belongs.

A "biomarker" is anything that can be used as an indicator of a particular disease state or some other physiological state of an organism, such as a mammal or a human. A biomarker can be the presence or absence of a gene, measure of gene expression, presence or absence of a protein, measure of protein expression or functional effect of the protein activity that can be measured and correlated with a physiological state. Biomarkers are used in medicine as laboratory parameters that a physician can use to help make decisions in making a diagnosis and selecting a course of treatment. Moreover, biomarkers, as is typically and preferably the case for the biomarkers of the present invention, are used to help optimize ideal treatments and indicates the likelihood of benefiting from a specific therapy. The preferred biomarker of the present invention are described throughout the specification and appended claims.

The term "status of a biomarker", as used herein, should refer to a status of a biomarker that is correlated with vulnerability to the PI3K inhibitor, preferably to the PI3K/mTOR inhibitor in accordance with the present invention. Thus, by identifying the status of a biomarker and comparing it to the normal status of said biomarker, it can be determined whether a mammal or human patient's SCC is more likely to be vulnerable to the PI3K inhibitor, preferably to the PI3K/mTOR inhibitor therapy of the present invention, and, thus, whether the mammal or human patient is a good responder or responder that will benefit from said therapy, or, to the contrary, a poor responder or non-responder that will not benefit or will have little benefit from said therapy.

Thus, the term "normal status" of a biomarker can denote a normal status of such biomarker, which corresponds to the status of the biomarker which, typically and preferably, correspond to the status of such biomarker in a healthy mammal or human patient, specifically such as the wild-type DNA *NOTCH1* sequence, or can denote a normal status and level, respectively, cleaved NOTCH1 protein (NICD1). Thus, by comparing the status of a biomarker of a mammal or human patient's tumor material or sample with its normal status, it can be determined, whether a mammal or human patient's tumor material or sample and thus a mammal or human patient is likely to benefit from said PI3K inhibitor, preferably to said PI3K/mTOR inhibitor therapy in accordance with the present invention. The "normal status" can, thus, refer to the sequence, parameter or level, typically and preferably, measured for comparison in a non-cancerous, healthy, wild-type tissue or cell, or in particular embodiment, placebo treated tumor cell.

The term "tumor" as used herein, should not be limited to a said primary tumor but typically and preferably include any tumor cell or group of cells that has moved away from the primary tumor. **In** the metastatic setting, the "tumor" of a said mammal or human patient may be localized in numerous different sites of the mammal's or human patient's body. Unless stated otherwise, when used in the form of "the tumor of a patient", the term refers to all tumor cells in the said mammal or human patient's body.

The term "squamous cell carcinoma" abbreviated as "SCC", as used herein, should typically and preferably include any SCC cell or group of cells that has moved away from the primary SCC site. **In** the metastatic setting, the "SCC" of a said mammal or human patient may be localized in numerous different sites of the mammal's or human patient's body. Unless stated otherwise, when used in the form of "the SCC of a patient", the term refers to all SCC cells in the said mammal or human patient's body.

The term "head and neck squamous cell carcinoma" abbreviated as "HNSCC", as used herein, should typically and preferably include any HNSCC cell or group of cells that has moved away from the primary HNSCC site. **In** the metastatic setting, the "HNSCC" of a said mammal or human patient may be localized in numerous different sites of the mammal's or human patient's body. Unless stated otherwise, when used in the form of "the HNSCC of a patient", the term refers to all HNSC cells in the said mammal or human patient's body.

The term "tumor material", as used herein, should refer to any material, such as, typically and preferably, any group of cells, any cell, or any sub-cellular component, DNA, mRNA, protein or product secreted therefrom, that originates from a said tumor, said SCC, and/or said HNSCC as defined herein, regardless of the method by which it was collected. The methods of collection of said tumor material are known to the skilled person in the art.

The term "sequenced DNA" as used herein, should refer to DNA obtained by sequencing methods known to the skilled person such as next generation sequencing but further should include cell-free circulating tumor DNA (ctDNA). "Next generation sequencing" methods are a group of high-throughput sequencing methods that parallelize the sequencing process, producing thousands or millions of sequences at once. The combination of the increase in data generated, coupled with lowered costs required to generate these data, has made this technology be recognized by those of skill in the art as a tractable, general purpose tool. Although a primary tumor itself or metastases derived from a primary tumor are currently the main source of tumor material including tumor DNA, acquiring tumor DNA through a biopsy is invasive, risky and often not possible. Dying tumor cells release small pieces of their DNA via different mechanisms into the bloodstream. These pieces are called cell-free circulating tumor DNA (ctDNA). In other words, ctDNA is tumor-derived fragmented DNA in the bloodstream that is not associated with cells. Because ctDNA may reflect the tumor genome in a more comprehensive manner, it has gained traction for its potential clinical utility. ctDNA can be isolated from different body fluids of a person, commonly referred to liquid biopsies. At present, plasma (derived from blood) is most commonly used as a source for ctDNA, but other body fluids including but not limited to saliva, urine and cerebrospinal fluid may also contain ctDNA.

The term "vulnerability of a squamous cell carcinoma (SCC)" as used herein, should in particular refer to the prediction that selected mammals or human SCC patients are predicted to likely benefit from therapeutic administration of the PI3K inhibitor, preferably PI3K/mTOR inhibitor, most preferably of the administration of bimiralisib, because the SCC is sensitive and susceptible to the PI3K inhibitor, preferably PI3K/mTOR inhibitor therapy, including but not limited due to increased growth arrest and/or increased apoptosis believed to be caused by the loss of function in accordance with the present invention of the NOTCH1 protein. A "loss of function" (LoF) mutation, as used herein, is a mutation in the DNA of a gene, the result of which is that the gene product (such as the encoded protein) has less than normal or no function in a cell or organism (including a human cell or human being).

The term "recurrent head and neck squamous cell carcinoma", as used herein, refers to a head and neck squamous cell carcinoma (HNSCC), which had disappeared in response to a previous treatment but subsequently recurred.

The term "metastatic head and neck squamous cell carcinoma", as used herein, refers to a head and neck squamous cell carcinoma (HNSCC), which has spread to other sites within the body, forming so-called metastases.

The term "*NOTCH1* loss-of-function (LoF) mutation", as used herein, refers to any genetic mutation in the *NOTCH1* gene in accordance with the present invention which are considered to result in loss of function of the NOTCH1 protein.

The term "an amount incompatible with NOTCH1 loss-of-function" as used herein, in particular in relation to the presence of NICD1, refers to an amount of NICD1 that is normally physiologically translocated into the nucleus following activation of NOTCH1 at the plasma membrane.

The terms "cleaved NOTCH1 intracellular domain protein", "cleaved NOTCH1 protein", "cl- NOTCH1 protein", "cl-NOTCH1" and NICD1", are interchangeably used herein.

The term "treating" as used herein, unless otherwise indicated, means reversing, alleviating, inhibiting the progress of the growth of tumors, tumor metastases, or other cancer-causing or neoplastic cells in a mammal or a human patient. The term "treatment" as used herein, unless otherwise indicated, refers to the act of treating.

The phrase "a method of treating" or its equivalent, when applied to cancer treatment, refers to a procedure or course of action that is designed to reduce or eliminate the number of cancer cells or to alleviate the symptoms of a cancer. "A method of treating" cancer or another proliferative disorder does not necessarily mean that the cancer cells or other disordered cells will actually be eliminated, that the number of cells or disorder will actually be reduced, or that the symptoms of a cancer or other disorder will actually be alleviated. Often, a method of treating cancer will be performed even with a low likelihood of success, but which, given the medical history and estimated survival expectancy, is nevertheless deemed an overall beneficial course of action.

A "therapeutically effective amount" or "effective amount" is the amount of a PI3K inhibitor, preferably a PI3K/mTOR inhibitor, in particular of the very preferred PI3K/mTOR inhibitor named bimiralisib in accordance with the present invention that will elicit the biological or medical response of a tumor material, mammal or human patient that is being sought by the researcher, veterinarian, medical doctor or other clinician. The term "therapeutic administration", as used herein, should refer to the administration of therapeutically effective amount.

A "pharmaceutical composition" is a combination of active agent and another carrier, e.g., compound or composition, inert (for example, a detectable agent or label) or active, such as an adjuvant, diluent, binder, stabilizer, buffers, salts, lipophilic solvents, preservative, adjuvant or the like. Carriers also include pharmaceutical excipients and additives, for example; proteins, peptides, amino acids, lipids, and carbohydrates (e.g., sugars, including monosaccharides and oligosaccharides; derivatized sugars such as alditols, aldonic acids, esterified sugars and the like; and polysaccharides or sugar polymers), which can be present singly or in combination, comprising alone or in combination 1-99.99% by weight or volume. Carbohydrate excipients include, for example; monosaccharides such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, sorbitol (glucitol) and myoinositol. It can be solid or in a liquid form.

A "substitution" is a mutation that exchanges one base for another (i.e., a change in a single "chemical letter" such as switching an A to a G). Such a substitution could (i) change a codon to one that encodes a different amino acid and cause a small change in the protein produced; (ii) change a codon to one that encodes the same amino acid and causes no change in the protein produced ("silent mutations"); or (iii) change an amino-acid-coding codon to a single "stop" codon and cause an incomplete protein (an incomplete protein is usually nonfunctional). An "insertion" is a mutation in which one or multiple extra base pairs are inserted into a place in the DNA. A "deletion" is a mutation in which a one or multiple base pairs or a section of DNA is lost, or deleted.

A "splice site mutation" is a genetic mutation that inserts or deletes a number of nucleotides in the specific site at which splicing of an intron takes place during the processing of precursor messenger RNA into mature messenger RNA. The abolishment of the splicing site results in one or more introns remaining in mature mRNA and may lead to the production of aberrant proteins.

An "in-frame mutation" or a "frameshift mutation", which terms are interchangeably used herein, is a mutation caused by insertions or deletions of a number of nucleotides that is not evenly divisible by three from a DNA sequence. Due to the triplet nature of gene expression by codons, the insertion or deletion can change the reading frame (the grouping of the codons), resulting in a completely different translation from the original. This often generates truncated proteins that result in loss of function.

A "missense mutation" is a point mutation where a single nucleotide is changed to cause substitution of a different amino acid. Missense mutation is a type of nonsynonymous substitution in a DNA sequence. Missense mutations can render the resulting protein nonfunctional, however, not all missense mutations lead to appreciable protein changes. An amino acid may be replaced by an amino acid of very similar chemical properties, in which case, the protein may still function normally; this is termed a neutral, "quiet", "silent" or "conservative mutation". A "nonsense mutation", in turn, is another type of nonsynonymous substitution in which a codon is changed to a premature stop codon that results in truncation of the resulting protein.

The term "a missense or an in-frame mutation incompatible with NOTCH1 loss-of-function" as used herein should refer to a missense or an in-frame mutation which mutation either does not cause truncation or which mutation would still lead to a functional protein, and thus said later mutation would be, for example, a silent or conservative missense mutation.

Therefore, in a preferred embodiment of the present invention and of all aspects of the present invention, said missense or an in-frame mutation incompatible with NOTCH1 loss-of-function is a missense or an in-frame mutation (i) not causing truncation of the resulting protein, preferably of a protein corresponding to human NOTCH1 protein of SEQ ID NO:2, or (ii) leading to a functional protein, preferably to a functional protein corresponding to human NOTCH1 protein of SEQ ID NO:2.

In a further very preferred embodiment of the present invention and of all aspects of the present invention, said missense or an in-frame mutation incompatible with NOTCH1 loss-of-function is a missense or an in-frame mutation not causing truncation of the resulting protein, preferably of a protein corresponding to human NOTCH1 protein of SEQ ID NO:2.

Thus, in a first aspect, the present invention provides a method of predicting the vulnerability of a squamous cell carcinoma (SCC) to inhibition by a PI3K inhibitor, preferably by a PI3K/mTOR inhibitor, wherein said method comprises
(a) identifying the status of a biomarker from tumor material from a mammal, preferably from a human patient, wherein the biomarker is selected from the group consisting of
   (i) sequenced tumor DNA, preferably human tumor DNA, to identify one or more mutations in the *NOTCH1* gene, preferably in the human *NOTCH1* gene of SEQ ID NO:1, encoding the NOTCH1 protein, preferably the human NOTCH1 protein of SEQ ID NO:2;
   (ii) protein level of cleaved NOTCH1 intracellular domain, preferably the protein level of human cleaved NOTCH1 intracellular domain (NICD1; SEQ ID NO:3); and
   (iii) a combination of biomarker (i) and (ii);
(b) comparing the status of the biomarker in said tumor material to a normal status of the biomarker; and
(c) selecting the mammal, preferably the human patient, as being predicted to benefit from therapeutic administration of the PI3K inhibitor, preferably of the PI3K/mTOR inhibitor, if
   (i) said mammal's, preferably human patient's, SCC harbors one or more *NOTCH1* mutations, wherein said *NOTCH1* mutation is not
      a. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2;
      b. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; or
      c. a mutation in the splice donor boundary (Exon 33), or in the acceptor boundary (Exon 34) of said NOTCH1 gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO: 1; or
   (ii) said mammal's, preferably human patient's, SCC comprises cleaved NOTCH1 intracellular domain protein, preferably human cleaved NOTCH1 intracellular domain (NICD1; SEQ ID NO:3) in an amount incompatible with NOTCH1 loss-of-function; or
   (iii) a combination of (i) and (ii).

The Cancer Genome Atlas (TCGA) describes nearly 100 *NOTCH1* mutations identified for T-ALL and HNSCC with the pattern, site of occurrence and nature of said *NOTCH1* mutations (Cancer Genome Atlas 2015, Nowell and Radtke 2017). The comparison of the pattern of said *NOTCH1* mutations led us to conclude the oncogenic *NOTCH1* mutations found in T-ALL occur in a hotspot of mostly missense mutations within the negative regulatory HD domain or in a second hotspot of mostly truncating mutations near the C-terminus, deleting the PEST domain and causing increased stabilization of activated *NOTCH1* in the nucleus (Ferrando 2009, Nowell and Radtke 2017). However, in HNSCC the truncating mutations are scattered throughout the protein but not in the PEST domain, and the missense mutations cluster in the extracellular EGF-ligand binding domains. Unlike T-ALL, only 1% of *NOTCH1* missense mutations in HNSCC are in either the negative regulatory or PEST domains. Without being bound, it is believed that mutations in the splice donor boundary (Exon 33), or in the acceptor boundary (Exon 34) of said *NOTCH1* gene are able to truncate said *NOTCH1* gene, in particular, said human *NOTCH1* gene of SEQ ID NO:1, within said TAD domain or said PEST domain.

In a further aspect, the present invention provides a method of predicting the vulnerability of a tumor material from a squamous cell carcinoma (SCC) of a mammal, preferably of a human patient, to a PI3K inhibitor, preferably a PI3K/mTOR inhibitor, wherein said method comprises contacting a tumor material from a squamous cell carcinoma (SCC) with said PI3K inhibitor, preferably said PI3K/mTOR inhibitor, and
(a) identifying the status of a biomarker of said tumor material, wherein the biomarker is selected from the group consisting of
   (i) sequenced tumor DNA, preferably human tumor DNA, to identify one or more mutations in the *NOTCH1* gene, preferably in the human *NOTCH1* gene of SEQ ID NO:1 , encoding the NOTCH1 protein, preferably the human NOTCH1 protein of SEQ ID NO:2 ;
   (ii) protein level of cleaved NOTCH1 intracellular domain, preferably the protein level of human cleaved NOTCH1 intracellular domain (NICD1; SEQ ID NO:3); and
   (iii) a combination of biomarker (i) and (ii);
(b) comparing the status of the biomarker in said tumor material to a normal status of the biomarker; and
(c) determining the vulnerability of the tumor material to a PI3K inhibitor, preferably of a PI3K/mTOR inhibitor based on the difference of the status of the biomarker in the tumor material and the normal status, and wherein determining the tumor material of said mammal, preferably said human patient, as being vulnerable to a PI3K inhibitor, preferably of a PI3K/mTOR inhibitor, if
   (i) said mammal's, preferably human patient's, tumor material harbors one or more *NOTCH1* mutations, wherein said *NOTCH1* mutation is not
      a. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2;
      b. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; or
      c. a mutation in the splice donor boundary (Exon 33), or in the acceptor boundary (Exon 34) of said NOTCH1 gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO: 1; or
   (ii) said mammal's, preferably human patient's, tumor material comprises cleaved NOTCH1 intracellular domain protein, preferably human cleaved NOTCH1 intracellular domain (NICD1; SEQ ID NO:3) in an amount incompatible with NOTCH1 loss-of-function; or
   (iii) a combination of (i) and (ii).

In another aspect, the present invention provides a method of method of treating a squamous cell carcinoma (SCC) of a mammal, preferably a human patient, comprising administering a therapeutically effective amount of a PI3K inhibitor, preferably a therapeutically effective amount of a PI3K/mTOR inhibitor to said mammal, preferably said human patient, wherein
(i) said mammal's, preferably human patient's, SCC harbors one or more *NOTCH1* mutations, wherein said *NOTCH1* mutation is not
   a. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2;
   b. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; or
   c. a mutation in the splice donor boundary (Exon 33), or in the acceptor boundary (Exon 34) of said *NOTCH1* gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1; or
(ii) said mammal's, preferably human patient's, SCC comprises cleaved NOTCH1 intracellular domain protein, preferably human cleaved NOTCH1 intracellular domain (NICD1; SEQ ID NO:3) in an amount incompatible with NOTCH1 loss-of-function; or
(iii) a combination of (i) and (ii).

In another aspect, the present invention provides a method of method of treating a squamous cell carcinoma (SCC) of a mammal, preferably a human patient with a PI3K inhibitor, preferably a PI3K/mTOR inhibitor, wherein said method comprises
(a) selecting said mammal, preferably said human patient, as being predicted to benefit from said treatment with said PI3K inhibitor, preferably said PI3K/mTOR inhibitor, wherein said selecting comprises
   (i) identifying the status of a biomarker from tumor material from said mammal, preferably from said human patient, wherein the biomarker is selected from the group consisting of
      a. sequenced tumor DNA, preferably human tumor DNA, to identify one or more mutations in the *NOTCH1* gene, preferably in the human *NOTCH1* gene of SEQ ID NO:1, encoding the NOTCH1 protein, preferably the human NOTCH1 protein of SEQ ID NO:2;
      b. protein level of cleaved NOTCH1 intracellular domain, preferably the protein level of human cleaved NOTCH1 intracellular domain (NICD1; SEQ ID NO:3); and
      c. a combination of biomarker (i) and (ii);
   (ii) comparing the status of the biomarker in said tumor material to a normal status of the biomarker; and
   (iii) selecting the mammal, preferably the human patient, as being predicted to benefit from said treatment with said PI3K inhibitor, preferably said PI3K/mTOR inhibitor, if
      a. said mammal's, preferably human patient's, SCC harbors one or more *NOTCH1* mutations, wherein said *NOTCH1* mutation is not
         i. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2;
         ii. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2 (full length human NOTCH1 protein]
         iii. a mutation in the splice donor boundary (Exon 33), or in the acceptor boundary (Exon 34) of said *NOTCH1* gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1; or
      b. said mammal's, preferably human patient's, SCC comprises cleaved NOTCH1 intracellular domain protein, preferably human cleaved NOTCH1 intracellular domain (NICD1; SEQ ID NO:3) in an amount incompatible with NOTCH1 loss-of-function; or
      c. a combination of a. and b.;
(b) administering a therapeutically effective amount of said PI3K inhibitor, preferably a therapeutically effective amount of said PI3K/mTOR inhibitor to said selected mammal, preferably said selected human patient.

In accordance with the present invention, SCCs considered to harbor a LoF mutation in the *NOTCH1* gene are more likely to respond to (i.e., to shrink due to believed increased apoptosis) a PI3K inhibitor, preferably to a PI3K/mTOR inhibitor, in particular to bimiralisib. Detection of one or more of said LoF mutations in the *NOTCH1* gene predicts that the patient will benefit from treatment with the PI3K inhibitor, preferably PI3K/mTOR inhibitor, and in particular with bimiralisib.

In an embodiment of the present invention, said identifying the status of a biomarker from tumor material from a mammal, preferably from a human patient, comprise providing the status of said biomarker of said tumor material such as, typically and preferably, the sequenced tumor DNA, preferably sequenced human tumor DNA, and/or the protein level of NICD1, typically and preferably by using appropriate assays. Typically and preferably, the status of said biomarker is provided by generating said sequenced tumor DNA by a sequencing assay, typically and preferably by a commercially available sequencing assay, and detecting said one or more mutations in the *NOTCH1* gene from the dataset generated by said sequencing assay. Thus, typically and preferably, the methods of the present invention may include but need not to include the steps such as the generating of said sequenced tumor DNA; the provision of the status of said biomarker of said tumor material which allows the identification of one or more mutations in the NOTCH1 gene and/or the protein level of NICD1 in accordance with the present invention is sufficient.

In an embodiment of the present invention, in particular for the method of predicting the vulnerability of a tumor material from a squamous cell carcinoma (SCC) of a mammal, preferably of a human patient, to a PI3K inhibitor, preferably a PI3K/mTOR inhibitor, said identifying the status of a biomarker of said tumor material comprise contacting a tumor material from a squamous cell carcinoma (SCC) with said PI3K inhibitor, preferably said PI3K/mTOR inhibitor, and/or further providing the status of said biomarker of said tumor material such as the sequenced tumor DNA, preferably sequenced human tumor DNA, and/or the protein level of NICD1, typically and preferably, by using appropriate assays such as by generating said sequenced tumor DNA by a sequencing assay, typically and preferably by a commercially available sequencing assay, and detecting said one or more mutations in the *NOTCH1* gene from the dataset generated by said sequencing assay. Thus, typically and preferably, the methods of the present invention, in particular the method of predicting the vulnerability of a tumor material from a squamous cell carcinoma (SCC) of a mammal, preferably of a human patient, to a PI3K inhibitor, preferably a PI3K/mTOR inhibitor, may include but need not to include the contacting step and/or the generating of said sequenced tumor DNA; the provision of the status of said biomarker of said tumor material which allows the identification of one or more mutations in the *NOTCH1* gene and/or the protein level of NICD1 in accordance with the present invention is sufficient.

In a preferred embodiment of the present invention, said NICD1 is determined by immunohistochemistry (IHC). In a further preferred embodiment of the present invention, said assay to measure said protein level of NICD1 is by immunohistochemistry (IHC), and wherein preferably said IHC assay is effected as described in the examples.

In a preferred embodiment of the present invention, said mammal is a mammal selected from the group consisting of a cat, a dog, a horse or a human, preferably a cat, a dog, or a human. In a further very preferred embodiment of the present invention, said mammal is a human patient.

In a further very preferred embodiment of the present invention, said mammal is a human patient, wherein for this very preferred embodiment of the present invention, the inventive methods thus refer, typically and preferably to the respective human genes and proteins.

Phosphoinositide 3-kinase (PI3K) inhibitors, preferably dual PI3K/mammalian target of rapamycin (mTOR) inhibitors are known for the skilled person in the art and have been described extensively (Thorpe, L. M., Yuzugullu, H. & Zhao, J. J. PI3K in cancer: divergent roles of isoforms, modes of activation and therapeutic targeting. (2015) Nat. Rev. Cancer 15, 7-24, WO2010/052569; WO2016/075130, the entire disclosure of which incorporated herein by way of reference). Preferred PI3K inhibitors, preferably PI3K/mTOR inhibitors, usable for the present invention, are disclosed in Table 2 of Thorpe et al. (2015) and are selected from the group of BKM120, GDC0941, BAY806946, ZSTK474, PX866, XL147, CH5132799, GDC0980, PF04691502, BGT226, BEZ235, XL765, GSK2126458, DS7423, PWT33597, SF1126, PF05212384, BAY806942, BYL719 and bimiralisib.

In a further preferred embodiment of the present invention, said PI3K inhibitor, preferably said PI3K/mTOR inhibitor is selected from the group consisting of BKM120, GDC0941, BAY806946, ZSTK474, PX866, XL147, CH5132799, GDC0980, PF04691502, BGT226, BEZ235, XL765, GSK2126458, DS7423, PWT33597, SF1126, PF05212384, BAY806942, BYL719 and bimiralisib. **In** a further preferred embodiment of the present invention, said PI3K inhibitor, preferably said PI3K/mTOR inhibitor is selected from the group consisting of BKM120, GDC0980, BEZ235, GSK2126458, BAY806942, BYL719 and bimiralisib.

**In** a further preferred embodiment, of the present invention, said PI3K inhibitor, preferably said PI3K/mTOR inhibitor is selected from the group consisting of
5-(4,6-dimorpholino-1,3,5-triazin-2-yl)-4-(trifluoromethyl)pyridin-2-amine;
5-(4,6-dimorpholino-1,3,5-triazin-2-yl)-4-(trifluoromethyl)pyrimidin-2-amine;
5-(4-(3-oxa-8-azabicyclo[3.2.1]octan-8-yl)-6-(3-oxa-8-azabicyclo[3.2.1]octan-8-yl)-1,3,5-triazin-2-yl)-4-(difluoromethyl)pyridin-2-amine;
(S)-4-(difluoromethyl)-5-(4-(3-methylmorpholino)-6-morpholino-1,3,5-triazin-2-yl)pyridin-2-amine;
(S)-4-(difluoromethyl)-5-(4-(3-methylmorpholino)-6-morpholino-1,3,5-triazin-2-yl)pyrimidin-2-amine;
(R)-4-(difluoromethyl)-5-(4-(3-methylmorpholino)-6-morpholino-1,3,5-triazin-2-yl)pyridin-2-amine;
(R)-4-(difluoromethyl)-5-(4-(3-methylmorpholino)-6-morpholino-1,3,5-triazin-2-yl)pyrimidin-2-amine
4-(difluoromethyl)-5-(4,6-dimorpholino-1,3,5-triazin-2-yl)pyrimidin-2-amine;
5-(4-(3-oxa-8-azabicyclo[3.2.1]octan-8-yl)-6-morpholino-1,3,5-triazin-2-yl)-4-(difluoromethyl)pyridin-2-amine;
5-(4,6-bis((S)-3-methylmorpholino)-1,3,5-triazin-2-yl)-4-(difluoromethyl)pyrimidin-2-amine;
4-(difluoromethyl)-5-(4-morpholino-6-(piperazin-1-yl)-1,3,5-triazin-2-yl)pyrimidin-2-amine; and
4-(difluoromethyl)-5-(4,6-dimorpholino-1,3,5-triazin-2-yl)pyridin-2-amine.

In a further preferred embodiment, of the present invention, said PI3K inhibitor, preferably said PI3K/mTOR inhibitor is selected from the group consisting of BKM120, GDC0941, BAY806946, ZSTK474, PX866, XL147, CH5132799, GDC0980, PF04691502, BGT226, BEZ235, XL765, GSK2126458, DS7423, PWT33597, SF1126, PF05212384, BAY806942, BYL719; and
5-(4,6-dimorpholino-1,3,5-triazin-2-yl)-4-(trifluoromethyl)pyridin-2-amine;
5-(4,6-dimorpholino-1,3,5-triazin-2-yl)-4-(trifluoromethyl)pyrimidin-2-amine;
5-(4-(3-oxa-8-azabicyclo[3.2.1]octan-8-yl)-6-(3-oxa-8-azabicyclo[3.2.1]octan-8-yl)-
1,3,5-triazin-2-yl)-4-(difluoromethyl)pyridin-2-amine;
(S)-4-(difluoromethyl)-5-(4-(3-methylmorpholino)-6-morpholino-1,3,5-triazin-2-yl)pyridin-2-amine;
(S)-4-(difluoromethyl)-5-(4-(3-methylmorpholino)-6-morpholino-1,3,5-triazin-2-yl)pyrimidin-2-amine;
(R)-4-(difluoromethyl)-5-(4-(3-methylmorpholino)-6-morpholino-1,3,5-triazin-2-yl)pyridin-2-amine;
(R)-4-(difluoromethyl)-5-(4-(3-methylmorpholino)-6-morpholino-1,3,5-triazin-2-yl)pyrimidin-2-amine
4-(difluoromethyl)-5-(4,6-dimorpholino-1,3,5-triazin-2-yl)pyrimidin-2-amine;
5-(4-(3-oxa-8-azabicyclo[3.2.1]octan-8-yl)-6-morpholino-1,3,5-triazin-2-yl)-4-(difluoromethyl)pyridin-2-amine;
5-(4,6-bis((S)-3-methylmorpholino)-1,3,5-triazin-2-yl)-4-(difluoromethyl)pyrimidin-2-amine;
4-(difluoromethyl)-5-(4-morpholino-6-(piperazin-1-yl)-1,3,5-triazin-2-yl)pyrimidin-2-amine; and
4-(difluoromethyl)-5-(4,6-dimorpholino-1,3,5-triazin-2-yl)pyridin-2-amine.

In a further preferred embodiment, of the present invention, said PI3K inhibitor, preferably said PI3K/mTOR inhibitor is selected from any of the following formula

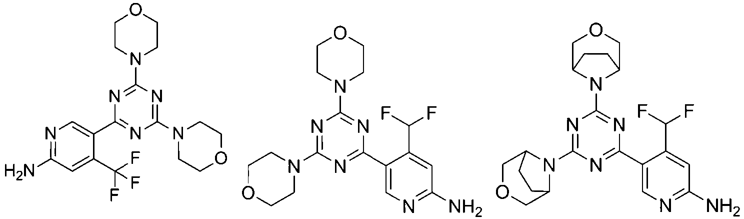

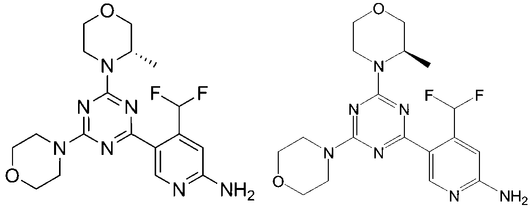

In a further very preferred embodiment of the present invention, said PI3K inhibitor, preferably said PI3K/mTOR inhibitor, is bimiralisib or a pharmaceutically acceptable salt thereof, wherein preferably said pharmaceutically acceptable salt is the tosylate of bimiralisib (Beaufils, Cmiljanovic et al. 2017, Bohnacker, Prota et al. 2017). Bimiralisib, also known as PQR309 or PQR-309, and identified by CAS No.: 1225037-39-7 has the following chemical structure:

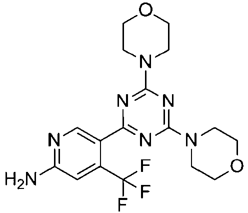

In a further very preferred embodiment of the present invention, said PI3K inhibitor, preferably said PI3K/mTOR inhibitor, is bimiralisib.

In a preferred embodiment of the present invention, said SCC is selected from the group consisting of head and neck squamous cell carcinoma (HNSCC), skin squamous cell carcinoma, esophagus squamous cell carcinoma, and lung squamous cell carcinoma. In another very preferred embodiment of the present invention, said SCC is head and neck squamous cell carcinoma (HNSCC). In a further preferred embodiment of the present invention, said SCC is recurrent or metastatic HNSCC. In a further preferred embodiment of the present invention, said SCC is recurrent HNSCC. In a further preferred embodiment of the present invention, said SCC is metastatic HNSCC. In a further preferred embodiment of the present invention, said SCC is recurrent and metastatic HNSCC.

In a preferred embodiment of the present invention, said biomarker is sequenced tumor DNA, preferably human tumor DNA, to identify one or more mutations in the *NOTCH1* gene, preferably in the human *NOTCH1* gene of SEQ ID NO:1, encoding the NOTCH1 protein, preferably the human NOTCH1 protein of SEQ ID NO:2. In a very preferred embodiment of the present invention, said mammal is a human patient, and said biomarker is sequenced human tumor DNA to identify one or more mutations in the human *NOTCH1* gene of SEQ ID NO:1, encoding the human NOTCH1 protein of SEQ ID NO:2.

In a very preferred embodiment of the present invention, in particular in a very preferred embodiment of the inventive method of predicting the vulnerability of a squamous cell carcinoma (SCC) to inhibition by a PI3K inhibitor, preferably by a PI3K/mTOR inhibitor, said selecting the mammal, preferably the human patient, as being predicted to benefit from therapeutic administration of the PI3K inhibitor, preferably of the PI3K/mTOR inhibitor, is if said mammal's, preferably human patient's, SCC harbors one or more *NOTCH1* mutations, wherein said *NOTCH1* mutation is not
a. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2; and is not
b. a missense or an in-frame mutation, preferably a missense or an in-frame mutation incompatible with NOTCH1 loss-of- function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; and is not
c. a mutation in the splice donor boundary (Exon 33), or in the acceptor boundary (Exon 34) of said *NOTCH1* gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1.

In a very preferred embodiment of the present invention, in particular in a very preferred embodiment of the inventive method of method of predicting the vulnerability of a tumor material from a squamous cell carcinoma (SCC) of a mammal, preferably of a human patient, to a PI3K inhibitor, preferably a PI3K/mTOR inhibitor, said determining the vulnerability of the tumor material to a PI3K inhibitor, preferably of a PI3K/mTOR inhibitor based on the difference of the status of the biomarker in the tumor material and the normal status, and wherein determining the tumor material of said mammal, preferably said human patient, as being vulnerable to a PI3K inhibitor, preferably of a PI3K/mTOR inhibitor, is if said mammal's, preferably human patient's, SCC harbors one or more *NOTCH1* mutations, wherein said *NOTCH1* mutation is not
a. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2; and is not
b. a missense or an in-frame mutation, preferably a missense or an in-frame mutation incompatible with NOTCH1 loss-of- function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; and is not
c. a mutation in the splice donor boundary (Exon 33), or in the acceptor boundary (Exon 34) of said NOTCH1 gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1.

In a very preferred embodiment of the present invention, in particular in a very preferred embodiment of the inventive method of treating a squamous cell carcinoma (SCC) of a mammal, preferably a human patient, wherein said mammal's, preferably human patient's, SCC harbors one or more *NOTCH1* mutations, wherein said *NOTCH1* mutation is not
a. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2; and is not
b. a missense or an in-frame mutation, preferably a missense or an in-frame mutation incompatible with NOTCH1 loss-of- function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; and is not
c. a mutation in the splice donor boundary (Exon 33), or in the acceptor boundary (Exon 34) of said NOTCH1 gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1.

In a very preferred embodiment of the present invention, in particular in a very preferred embodiment of the inventive method of treating a squamous cell carcinoma (SCC) of a mammal, preferably a human patient, said selecting the mammal, preferably the human patient, as being predicted to benefit from therapeutic administration of the PI3K inhibitor, preferably of the PI3K/mTOR inhibitor, is if said mammal's, preferably human patient's, SCC harbors one or more *NOTCH1* mutations, wherein said *NOTCH1* mutation is not
a. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2; and is not
b. a missense or an in-frame mutation, preferably a missense or an in-frame mutation incompatible with NOTCH1 loss-of- function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; and is not
c. a mutation in the splice donor boundary (Exon 33), or in the acceptor boundary (Exon 34) of said NOTCH1 gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1.

In a very preferred embodiment of the present invention, said mammal's, preferably human patient's, SCC harbors one or more *NOTCH1* mutations, wherein said *NOTCH1* mutation is not
a. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2; and is not
b. a missense or an in-frame mutation, preferably a missense or an in-frame mutation incompatible with NOTCH1 loss-of- function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; and is not
c. a mutation in the splice donor boundary (Exon 33), or in the acceptor boundary (Exon 34) of said NOTCH1 gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1.

In a very preferred embodiment of the present invention, said mammal is a human, and wherein said human patient's SCC harbors one or more *NOTCH1* mutations, wherein said *NOTCH1* mutation is not
a. a mutation in the TAD domain or in the PEST domain of said human *NOTCH1* gene corresponding to aa 2159-2555 of SEQ ID NO:2; and is not
b. a missense or an in-frame mutation, preferably a missense or an in-frame mutation incompatible with NOTCH1 loss-of- function, in the Lin-12/Notch 1 Repeats (LNR) or in the heterodimerization domain (HD domain) of said human *NOTCH1* gene corresponding to aa 1442-1734 of SEQ ID NO:2; and is not
c. a mutation in the splice donor boundary (Exon 33), or in the acceptor boundary (Exon 34) of said human *NOTCH1* gene corresponding to nt 5639-6082 of SEQ ID NO:1.

In a preferred embodiment of the present invention, said administration is an oral administration or a topical administration. In a further preferred embodiment of the present invention, said administration is an oral administration.

In a further very preferred embodiment of the present invention, said PI3K inhibitor, preferably said PI3K/mTOR inhibitor, and further preferably said bimiralisib, is formulated for oral administration, wherein preferably said PI3K inhibitor, preferably said bimiralisib is in the form of a tablet, a pill or a capsule, most preferably in the form of a capsule.

In a very preferred embodiment, the present invention provides a method of predicting the vulnerability of a squamous cell carcinoma (SCC) to inhibition by a PI3K inhibitor, preferably a PI3K/mTOR inhibitor, wherein said PI3K inhibitor is bimiralisib, wherein said method comprises
(a) identifying the status of a biomarker from tumor material from a human patient, wherein the biomarker is sequenced human tumor DNA to identify one or more mutations in the human *NOTCH1* gene of SEQ ID NO:1, encoding the human NOTCH1 protein of SEQ ID NO:2;
(b) comparing the status of the biomarker in said tumor material to a normal status of the biomarker; and
(c) selecting the human patient as being predicted to benefit from therapeutic administration of bimiralisib, if
   (i) said human patient's SCC harbors one or more *NOTCH1* mutations, wherein said *NOTCH1* mutation is not
      a. a mutation in the TAD domain or in the PEST domain of said *NOTCH1* gene corresponding to aa 2159-2555 of SEQ ID NO:2;
      b. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; or
      c. a mutation in the splice donor boundary (Exon 33), or in the acceptor boundary (Exon 34) of said human *NOTCH1* gene corresponding to nt 5639-6082 of SEQ ID NO:1.

Very preferably, said squamous cell carcinoma (SCC) is head and neck squamous cell carcinoma (HNSCC).

In a very preferred embodiment, the present invention provides a method of predicting the vulnerability of a tumor material from a squamous cell carcinoma (SCC) of a human patient, to a PI3K inhibitor, preferably a PI3K/mTOR inhibitor, wherein said PI3K inhibitor is bimiralisib, and wherein said method comprises contacting a tumor material from a squamous cell carcinoma (SCC) with bimiralisib, and
(a) identifying the status of a biomarker of said tumor material, wherein the biomarker is sequenced human tumor DNA to identify one or more mutations in the human *NOTCH1* gene of SEQ ID NO:1, encoding the human NOTCH1 protein of SEQ ID NO:2;
(b) comparing the status of the biomarker in said tumor material to a normal status of the biomarker; and
(c) determining the vulnerability of the tumor material to bimiralisib based on the difference of the status of the biomarker in the tumor material and the normal status, and wherein determining the tumor material of said human patient as being vulnerable to bimiralisib, if
   (i) said human patient's SCC harbors one or more *NOTCH1* mutations, wherein said *NOTCH1* mutation is not
      a. a mutation in the TAD domain or in the PEST domain of said *NOTCH1* gene corresponding to aa 2159-2555 of SEQ ID NO:2;
      b. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; or
      c. a mutation in the splice donor boundary (Exon 33), or in the acceptor boundary (Exon 34) of said human *NOTCH1* gene corresponding to nt 5639-6082 of SEQ ID NO:1.

Very preferably, said squamous cell carcinoma (SCC) is head and neck squamous cell carcinoma (HNSCC).

In a very preferred embodiment, the present invention provides a method of treating a squamous cell carcinoma (SCC) of a human patient, comprising administering a therapeutically effective amount of a PI3K inhibitor, preferably a therapeutically effective amount of a PI3K/mTOR inhibitor to said human patient, wherein said PI3K inhibitor is bimiralisib, and wherein
(i) said human patient's SCC harbors one or more *NOTCH1* mutations, wherein said *NOTCH1* mutation is not
   a. a mutation in the TAD domain or in the PEST domain of said *NOTCH1* gene corresponding to aa 2159-2555 of SEQ ID NO:2;
   b. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; or
   c. a mutation in the splice donor boundary (Exon 33), or in the acceptor boundary (Exon 34) of said human *NOTCH1* gene corresponding to nt 5639-6082 of SEQ ID NO:1.

Very preferably, said squamous cell carcinoma (SCC) is head and neck squamous cell carcinoma (HNSCC).

In a very preferred embodiment, the present invention provides a method of treating a squamous cell carcinoma (SCC) of a human patient with a PI3K inhibitor, preferably a PI3K/mTor inhibitor, wherein said PI3K inhibitor is bimiralisib, wherein said method comprises
(a) selecting said human patient as being predicted to benefit from said treatment with said bimiralisib, wherein said selecting comprises
   (i) identifying the status of a biomarker from tumor material from said human patient, wherein the biomarker is sequenced human tumor DNA to identify one or more mutations in the human *NOTCH1* gene of SEQ ID NO:1, encoding the human NOTCH1 protein of SEQ ID NO:2;
   (ii) comparing the status of the biomarker in said tumor material to a normal status of the biomarker; and
   (iii) selecting the human patient as being predicted to benefit from said treatment with said bimiralisib, if said human patient's, SCC harbors one or more *NOTCH1* mutations, wherein said *NOTCH1* mutation is not
      i. a mutation in the TAD domain or in the PEST domain of said *NOTCH1* gene corresponding to aa 2159-2555 of SEQ ID NO:2;
      ii. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; or
      iii. a mutation in the splice donor boundary (Exon 33), or in the acceptor boundary (Exon 34) of said human *NOTCH1* gene corresponding to nt 5639-6082 of SEQ ID NO:1;
(b) administering a therapeutically effective amount of said bimiralisib to said selected human patient.

Very preferably, said squamous cell carcinoma (SCC) is head and neck squamous cell carcinoma (HNSCC).

Thus, essentially in said very preferred embodiments, any mutations in the TAD or PEST domains, any missense or in-frame mutations in the LNR and HD domains, and splice donor (Exon 33) or acceptor (Exon 34) boundaries which would hypothetically truncate the protein within the TAD/PEST domains will be excluded as considered to benefit from the treatment in accordance with the present invention. All other *NOTCH1* mutations will be eligible. Thus, for said very preferred embodiments, human patients with *NOTCH1* mutations in regions associated with activation including TAD/PEST domains or mutations in LNR and HD domains that are not truncating would be excluded. Splice mutations in Exon 33 or 34 would also be excluded, but patients with *NOTCH1* mutation in all other regions would be eligible to benefit from the treatment in accordance with the present invention.

In another aspect, the present invention provides a kit for selecting a mammal, preferably a human patient, with squamous cell carcinoma being predicted to benefit or not to benefit from administration of a PI3K inhibitor, preferably of a PI3K/mTOR inhibitor, the kit comprising:
(a) a means for identifying in a tumor material a status of a biomarker selected from the group consisting of
   (i) sequenced tumor DNA, preferably human tumor DNA, to identify one or more mutations in the *NOTCH1* gene, preferably in the human *NOTCH1* gene of SEQ ID NO:1, encoding the NOTCH1 protein, preferably the human NOTCH1 protein of SEQ ID NO:2;
   (ii) protein level of cleaved NOTCH1 intracellular domain (cl-NOTCH1, [NICD1]; SEQ ID NO:3), wherein preferably said NICD1 is determined by immunohistochemistry (IHC); and
   (iii) a combination of biomarker (i) and (ii);
(b) a means for identifying the normal status.

In a preferred embodiment of the inventive kit, said means for identifying the normal status is information containing a predetermined normal status of the biomarker that has been correlated with vulnerability to the PI3K inhibitor, preferably to the PI3K/mTOR inhibitor.

In a further very preferred embodiment, said kit for selecting a mammal with squamous cell carcinoma being predicted to benefit or not to benefit from administration of a PI3K inhibitor, preferably of a PI3K/mTOR inhibitor, is a kit for selecting a human patient with squamous cell carcinoma, preferably HNSCC, being predicted to benefit or not to benefit from administration of said PI3K inhibitor, preferably of said PI3K/mTOR inhibitor, most preferably of bimilarisib.

In a further preferred embodiment of the inventive kit, said biomarker is sequenced tumor DNA, preferably sequenced human tumor DNA, and wherein the means for identifying the normal status is the wild-type human *NOTCH1* gene of SEQ ID NO:1.

In a further very preferred embodiment of the inventive kit, said kit is a kit for selecting a human patient with squamous cell carcinoma, preferably HNSCC, and said biomarker is sequenced human tumor DNA, and wherein the means for identifying the normal status is the wild-type human *NOTCH1* gene of SEQ ID NO:1.

In another aspect, the present invention provides a pharmaceutical composition comprising a therapeutically effective amount of a PI3K inhibitor, preferably a therapeutically effective amount of a PI3K/mTOR inhibitor for use in treatment of a squamous cell carcinoma (SCC) of a mammal, preferably a human patient, wherein
(i) said mammal's, preferably human patient's, SCC harbors one or more *NOTCH1* mutations, wherein said *NOTCH1* mutation is not
   a. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2;
   b. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; or
   c. a mutation in the splice donor boundary (Exon 33), or in the acceptor boundary (Exon 34) of said NOTCH1 gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1; or
(ii) said mammal's, preferably human patient's, SCC comprises cleaved NOTCH1 intracellular domain protein (cl-NOTCH1, [NICD1]; SEQ ID NO:3) in an amount incompatible with NOTCH1 loss-of-function; or
(iii) a combination of (i) and (ii).

In another aspect, the present invention provides a pharmaceutical composition comprising a therapeutically effective amount of a PI3K inhibitor, preferably a therapeutically effective amount of a PI3K/mTOR inhibitor for use in treatment of a squamous cell carcinoma (SCC) of a mammal, preferably a human patient, wherein said mammal, preferably said human patient is selected to benefit from said treatment with said PI3K inhibitor, preferably said PI3K/mTOR inhibitor, and wherein said selecting comprises
(i) identifying the status of a biomarker from tumor material from said mammal, preferably from said human patient, wherein the biomarker is selected from the group consisting of
   a. sequenced tumor DNA, preferably sequenced human tumor DNA, to identify one or more mutations in the *NOTCH1* gene, preferably in the human *NOTCH1* gene of SEQ ID NO:1, encoding the NOTCH1 protein, preferably the human NOTCH1 protein of SEQ ID NO:2;
   b. protein level of cleaved NOTCH1 intracellular domain (cl-NOTCH1, [NICD1]; SEQ ID NO:3), wherein preferably said NICD1 is determined by immunohistochemistry (IHC); and
   c. a combination of biomarker (i) and (ii);
(ii) comparing the status of the biomarker in said tumor material to a normal status of the biomarker; and
(iii) selecting the mammal, preferably the human patient, as being predicted to benefit from said treatment with said PI3K inhibitor, preferably said PI3K/mTOR inhibitor, if
   a. said mammal's, preferably human patient's, SCC harbors one or more *NOTCH1* mutations, wherein said *NOTCH1* mutation is not
      i. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2;
      ii. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2 (full length human NOTCH1 protein]
      iii. a mutation in the splice donor boundary (Exon 33), or in the acceptor boundary (Exon 34) of said NOTCH 1 gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1; or
   b. said mammal's, preferably human patient's, SCC comprises cleaved NOTCH1 intracellular domain protein (cl-NOTCH1, [NICD1]; SEQ ID NO:3) in an amount incompatible with NOTCH1 loss-of-function; or
   c. a combination of a. and b..

In a preferred embodiment of the inventive pharmaceutical composition, said mammal is a human patient, and wherein said squamous cell carcinoma (SCC) is head and neck squamous cell carcinoma (HNSCC), and wherein said PI3K inhibitor, preferably said PI3K/mTOR inhibitor, is bimiralisib.

The invention furthermore comprises the following items:
Item 1: A method of predicting the vulnerability of a squamous cell carcinoma (SCC) to inhibition by a PI3K inhibitor, preferably by a PI3K/mTOR inhibitor, wherein said method comprises
   (a) identifying the status of a biomarker from tumor material from a mammal, preferably from a human patient, wherein the biomarker is selected from the group consisting of
      (i) sequenced tumor DNA, preferably human tumor DNA, to identify one or more mutations in the *NOTCH1* gene, preferably in the human *NOTCH1* gene of SEQ ID NO:1, encoding the NOTCH1 protein, preferably the human NOTCH1 protein of SEQ ID NO:2;
      (ii) protein level of cleaved NOTCH1 intracellular domain, preferably the protein level of human cleaved NOTCH1 intracellular domain (NICD1; SEQ ID NO:3); and
      (iii) a combination of biomarker (i) and (ii);
   (b) comparing the status of the biomarker in said tumor material to a normal status of the biomarker; and
   (c) selecting the mammal, preferably the human patient, as being predicted to benefit from therapeutic administration of the PI3K inhibitor, preferably of the PI3K/mTOR inhibitor, if
      (i) said mammal's, preferably human patient's, SCC harbors one or more *NOTCH1* mutations, wherein said *NOTCH1* mutation is not
         a. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2;
         b. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; or
         c. a mutation in the splice donor boundary (Exon 33), or in the acceptor boundary (Exon 34) of said *NOTCH1* gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1; or
      (ii) said mammal's, preferably human patient's, SCC comprises cleaved NOTCH1 intracellular domain protein, preferably human cleaved NOTCH1 intracellular domain (NICD1; SEQ ID NO:3) in an amount incompatible with NOTCH1 loss-of-function; or
      (iii) a combination of (i) and (ii).
Item 2: A method of predicting the vulnerability of a tumor material from a squamous cell carcinoma (SCC) of a mammal, preferably of a human patient, to a PI3K inhibitor, preferably a PI3K/mTOR inhibitor, wherein said method comprises contacting a tumor material from a squamous cell carcinoma (SCC) with said PI3K inhibitor, preferably said PI3K/mTOR inhibitor, and
   (a) identifying the status of a biomarker of said tumor material, wherein the biomarker is selected from the group consisting of
      (i) sequenced tumor DNA, preferably human tumor DNA, to identify one or more mutations in the *NOTCH1* gene, preferably in the human *NOTCH1* gene of SEQ ID NO:1, encoding the NOTCH1 protein, preferably the human NOTCH1 protein of SEQ ID NO:2;
      (ii) protein level of cleaved NOTCH1 intracellular domain, preferably the protein level of human cleaved NOTCH1 intracellular domain (NICD1; SEQ ID NO:3); and
      (iii) a combination of biomarker (i) and (ii);
   (b) comparing the status of the biomarker in said tumor material to a normal status of the biomarker; and
   (c) determining the vulnerability of the tumor material to a PI3K inhibitor, preferably of a PI3K/mTOR inhibitor based on the difference of the status of the biomarker in the tumor material and the normal status, and wherein determining the tumor material of said mammal, preferably said human patient, as being vulnerable to a PI3K inhibitor, preferably of a PI3K/mTOR inhibitor, if
      (i) said mammal's, preferably human patient's, tumor material harbors one or more *NOTCH1* mutations, wherein said *NOTCH1* mutation is not
         a. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2;
         b. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; or
         c. a mutation in the splice donor boundary (Exon 33), or in the acceptor boundary (Exon 34) of said *NOTCH1* gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1; or
      (ii) said mammal's, preferably human patient's, tumor material comprises cleaved NOTCH1 intracellular domain protein, preferably human cleaved NOTCH1 intracellular domain (NICD1; SEQ ID NO:3) in an amount incompatible with NOTCH1 loss-of-function; or
      (iii) a combination of (i) and (ii).
Item 3: A method of treating a squamous cell carcinoma (SCC) of a mammal, preferably a human patient, comprising administering a therapeutically effective amount of a PI3K inhibitor, preferably a therapeutically effective amount of a PI3K/mTOR inhibitor to said mammal, preferably said human patient, wherein
   (i) said mammal's, preferably human patient's, SCC harbors one or more *NOTCH1* mutations, wherein said *NOTCH1* mutation is not
      a. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2;
      b. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; or
      c. a mutation in the splice donor boundary (Exon 33), or in the acceptor boundary (Exon 34) of said *NOTCH1* gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1; or
   (ii) said mammal's, preferably human patient's, SCC comprises cleaved NOTCH1 intracellular domain protein, preferably human cleaved NOTCH1 intracellular domain (NICD1; SEQ ID NO:3) in an amount incompatible with NOTCH1 loss-of-function; or
   (iii) a combination of (i) and (ii).
Item 4: A method of treating a squamous cell carcinoma (SCC) of a mammal, preferably a human patient with a PI3K inhibitor, preferably a PI3K/mTor inhibitor, wherein said method comprises
   (a) selecting said mammal, preferably said human patient, as being predicted to benefit from said treatment with said PI3K inhibitor, preferably said PI3K/mTOR inhibitor, wherein said selecting comprises
      (i) identifying the status of a biomarker from tumor material from said mammal, preferably from said human patient, wherein the biomarker is selected from the group consisting of
         a. sequenced tumor DNA, preferably human tumor DNA, to identify one or more mutations in the *NOTCH1* gene, preferably in the human *NOTCH1* gene of SEQ ID NO:1, encoding the NOTCH1 protein, preferably the human NOTCH1 protein of SEQ ID NO:2;
         b. protein level of cleaved NOTCH1 intracellular domain, preferably the protein level of human cleaved NOTCH1 intracellular domain (NICD1; SEQ ID NO:3); and
         c. a combination of biomarker (i) and (ii);
      (ii) comparing the status of the biomarker in said tumor material to a normal status of the biomarker; and
      (iii) selecting the mammal, preferably the human patient, as being predicted to benefit from said treatment with said PI3K inhibitor, preferably said PI3K/mTOR inhibitor, if
         a. said mammal's, preferably human patient's, SCC harbors one or more *NOTCH1* mutations, wherein said *NOTCH1* mutation is not
            i. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2;
            ii. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2 (full length human NOTCH1 protein]
            iii. a mutation in the splice donor boundary (Exon 33), or in the acceptor boundary (Exon 34) of said *NOTCH1* gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1; or
         b. said mammal's, preferably human patient's, SCC comprises cleaved NOTCH1 intracellular domain protein, preferably human cleaved NOTCH1 intracellular domain (NICD1; SEQ ID NO:3) in an amount incompatible with NOTCH1 loss-of-function; or
         c. a combination of a. and b.;
   (b) administering a therapeutically effective amount of said PI3K inhibitor, preferably a therapeutically effective amount of said PI3K/mTOR inhibitor to said selected mammal, preferably said selected human patient.
Item 5: The method of any of the preceding items, wherein said mammal is a human patient.
Item 6: The method of any of the preceding items, wherein said PI3K inhibitor, preferably said PI3K/mTOR inhibitor, is bimiralisib.
Item 7: The method of any of the preceding items, wherein said squamous cell carcinoma (SCC) is head and neck squamous cell carcinoma (HNSCC).
Item 8: The method of any of the preceding items, wherein said biomarker is sequenced tumor DNA, preferably human tumor DNA, to identify one or more mutations in the *NOTCH1* gene, preferably in the human *NOTCH1* gene of SEQ ID NO:1, encoding the NOTCH1 protein, preferably the human NOTCH1 protein of SEQ ID NO:2.
Item 9: The method of any of the preceding items, wherein said mammal is a human, and wherein said biomarker is sequenced human tumor DNA to identify one or more mutations in the human *NOTCH1* gene of SEQ ID NO:1, encoding the human NOTCH1 protein of SEQ ID NO:2.
Item 10: The method of any of the preceding items, wherein said mammal's, preferably human patient's, SCC harbors one or more *NOTCH1* mutations, wherein said *NOTCH1* mutation is not
   a. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2; and is not
   b. a missense or an in-frame mutation, preferably a missense or an in-frame mutation incompatible with NOTCH1 loss-of- function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; and is not
   c. a mutation in the splice donor boundary (Exon 33), or in the acceptor boundary (Exon 34) of said *NOTCH1* gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1.
Item 11: The method of any of the preceding items, wherein said mammal is a human, and wherein said human patient's SCC harbors one or more *NOTCH1* mutations, wherein said *NOTCH1* mutation is not
   a. a mutation in the TAD domain or in the PEST domain of said human *NOTCH1* gene corresponding to aa 2159-2555 of SEQ ID NO:2; and is not
   b. a missense or an in-frame mutation, preferably a missense or an in-frame mutation incompatible with NOTCH1 loss-of- function, in the Lin-12/Notch 1 Repeats (LNR) or in the heterodimerization domain (HD domain) of said human *NOTCH1* gene corresponding to aa 1442-1734 of SEQ ID NO:2; and is not
   c. a mutation in the splice donor boundary (Exon 33), or in the acceptor boundary (Exon 34) of said human *NOTCH1* gene corresponding to nt 5639-6082 of SEQ ID NO:1.
Item 12: A kit for selecting a mammal, preferably a human patient, with squamous cell carcinoma being predicted to benefit or not to benefit from administration of a PI3K inhibitor, preferably of a PI3K/mTOR inhibitor, the kit comprising:
   (a) a means for identifying in a tumor material a status of a biomarker selected from the group consisting of
      (i) sequenced tumor DNA, preferably human tumor DNA, to identify one or more mutations in the *NOTCH1* gene, preferably in the human *NOTCH1* gene of SEQ ID NO:1, encoding the NOTCH1 protein, preferably the human NOTCH1 protein of SEQ ID NO:2;
      (ii) protein level of cleaved NOTCH1 intracellular domain (cl-NOTCH1, [NICD1]; SEQ ID NO:3), wherein preferably said NICD1 is determined by immunohistochemistry (IHC); and
      (iii) a combination of biomarker (i) and (ii);
   (b) a means for identifying the normal status.
Item 13: A pharmaceutical composition comprising a therapeutically effective amount of a PI3K inhibitor, preferably a therapeutically effective amount of a PI3K/mTOR inhibitor for use in treatment of a squamous cell carcinoma (SCC) of a mammal, preferably a human patient, wherein
   (i) said mammal's, preferably human patient's, SCC harbors one or more *NOTCH1* mutations, wherein said *NOTCH1* mutation is not
      a. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2;
      b. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; or
      c. a mutation in the splice donor boundary (Exon 33), or in the acceptor boundary (Exon 34) of said *NOTCH1* gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1; or
   (ii) said mammal's, preferably human patient's, SCC comprises cleaved NOTCH1 intracellular domain protein (cl-NOTCH1, [NICD1]; SEQ ID NO:3) in an amount incompatible with NOTCH1 loss-of-function; or
   (iii) a combination of (i) and (ii).
Item 14: A pharmaceutical composition comprising a therapeutically effective amount of a PI3K inhibitor, preferably a therapeutically effective amount of a PI3K/mTor inhibitor for use in treatment of a squamous cell carcinoma (SCC) of a mammal, preferably a human patient, wherein said mammal, preferably said human patient is selected to benefit from said treatment with said PI3K inhibitor, preferably said PI3K/mTOR inhibitor, and wherein said selecting comprises
   (i) identifying the status of a biomarker from tumor material from said mammal, preferably from said human patient, wherein the biomarker is selected from the group consisting of
      a. sequenced tumor DNA, preferably sequenced human tumor DNA, to identify one or more mutations in the *NOTCH1* gene, preferably in the human *NOTCH1* gene of SEQ ID NO:1, encoding the NOTCH1 protein, preferably the human NOTCH1 protein of SEQ ID NO:2;
      b. protein level of cleaved NOTCH1 intracellular domain (cl-NOTCH1, [NICD1]; SEQ ID NO:3), wherein preferably said NICD1 is determined by immunohistochemistry (IHC); and
      c. a combination of biomarker (i) and (ii);
   (ii) comparing the status of the biomarker in said tumor material to a normal status of the biomarker; and
   (iii) selecting the mammal, preferably the human patient, as being predicted to benefit from said treatment with said PI3K inhibitor, preferably said PI3K/mTOR inhibitor, if
      a. said mammal's, preferably human patient's, SCC harbors one or more *NOTCH1* mutations, wherein said *NOTCH1* mutation is not
         i. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2;
         ii. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2 (full length human NOTCH1 protein]
         iii. a mutation in the splice donor boundary (Exon 33), or in the acceptor boundary (Exon 34) of said *NOTCH1* gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1; or
      b. said mammal's, preferably human patient's, SCC comprises cleaved NOTCH1 intracellular domain protein (cl-NOTCH1, [NICD1]; SEQ ID NO:3) in an amount incompatible with NOTCH1 loss-of-function; or
      c. a combination of a. and b..
Item 15: The pharmaceutical composition of item 13 or item 14, wherein said mammal is a human patient, and wherein said squamous cell carcinoma (SCC) is head and neck squamous cell carcinoma (HNSCC), and wherein said PI3K inhibitor, preferably said PI3K/mTOR inhibitor, is bimiralisib.

### EXAMPLES

The detailed description provided herein including within this example section is to illustrate the invention but not to limit its scope. Other variants of the invention will be readily apparent to one of ordinary skill in the art and are encompassed by the appended claims

### EXAMPLE 1

### Testing of efficacy of the preferred dual PI3K/mTOR inhibitor bimiralisib in a panel of 69 HNSCC cell lines

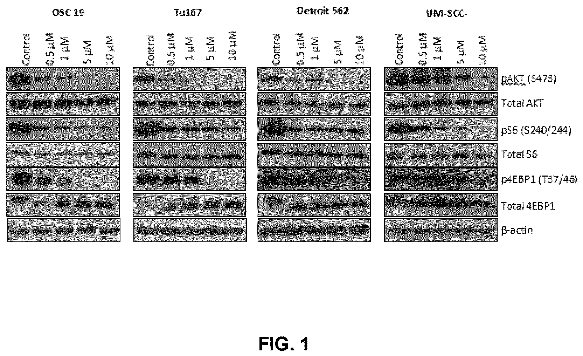

We observed a decrease in pAKT, pS6 and p4EBP1 upon treatment with bimiralisib (FIG. 1). We tested 69 HNSCC cell lines for sensitivity to bimiralisib and found that the majority were sensitive and 66 cell lines had IC₅₀ values < 3 µM and 36 cell lines had IC₇₀ values < 3 µM. To identify potential biomarkers of response to bimiralisib, we compared the drug sensitivity to baseline gene and protein expression as well as gene mutations. We found 3 proteins whose expression correlated with sensitivity to bimiralisib: CHK2, caveolin, and E2F1 while the sensitivity did not correlate with mutations in *TP53, CDKN2A, CASP8, HRAS, PIK3CA*)*, FAT1, AJUBA, FBXW7, KRAS, MAML.* However, the sensitivity did best correlate with the presence of a *NOTCH1* mutation (Table 1) indicating that there is sensitivity to bimiralisib in cells having *NOTCH1* mutations.

IC50 values were estimated from the best-fit dose-response model selected by calculating residual standard error using the R packages Dose Finding and drc (dose response curve) (Ritz and Streibig 2005, Bomkamp, Bretz et al. 2011). All experiments were done in duplicate and we compared the response between two experiments using concordance correlation coefficient (CCC). The CCC can be computed based on the scaled response as well as original unscaled response. The scaled version should be more relevant since this is actually the data used in IC estimation. Gene expression data were available for 49 of the 69 cell lines treated with bimiralisib. Reverse phase protein array (RPPA) data were obtained as previously described (Byers, Wang et al. 2012, Byers, Diao et al. 2013, Akbani, Ng et al. 2014) and available for 62 lines tested with bimiralisib. To identify differentially expressed features between the comparative groups, we applied modified two-sample t-tests using the Limma package. The beta-uniform mixture (BUM) model, described by Pounds and Morris was used to control false discovery rate (FDR) (Pounds and Morris 2003).

**Table 1: Bimiralisib sensitivity and NOTCH1 Mutations**

| | Sensitive | Resistant |
|---|---|---|
| WT | 18 | 20 |
| Mutant | 12 | 3 |

| | | |
|---|---|---|
| P = 0.0368 | | |

### EXAMPLE 2

### Exome sequencing (WES) on 66 established HNSCC lines

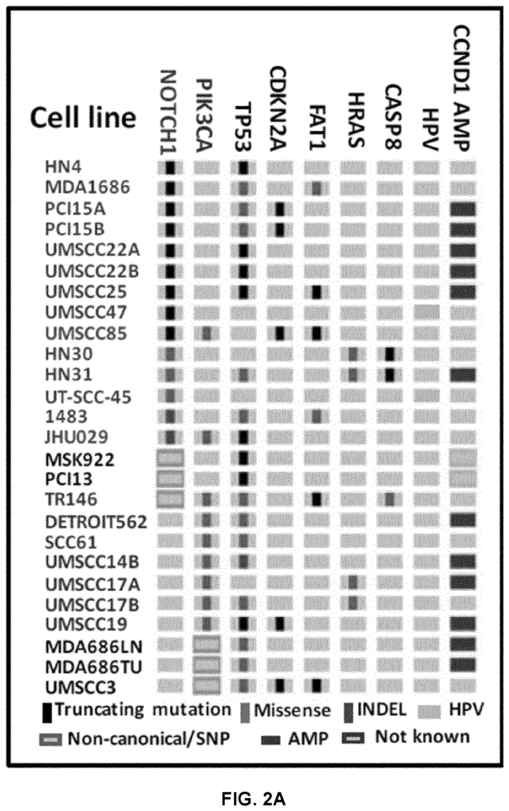

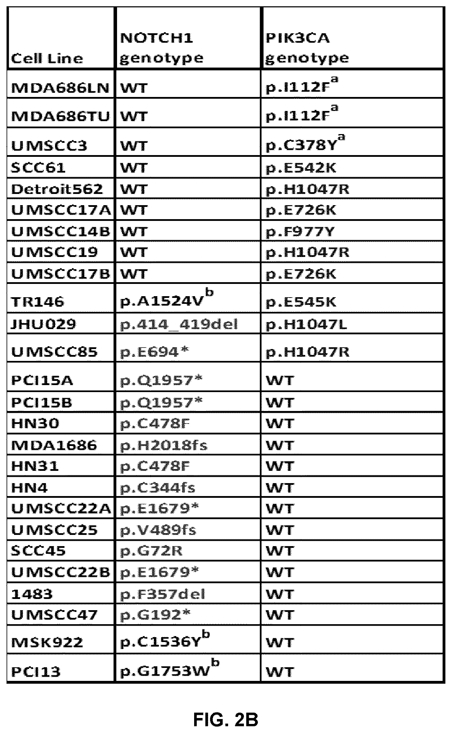

To address the function of *NOTCH1* mutations and other genes frequently altered in HNSCC, we performed whole exome sequencing (WES) on 66 established HNSCC lines. A number of these cell lines were mutant for *NOTCH1* or had *PIK3CA* mutations in known hotspots, but they also had additional driver mutations frequently observed in HNSCC (FIG. 2) and were therefore reflective of genomic subtypes found in patients. Most *NOTCH1* mutations in the HNSCC cell lines were truncating, and loss of NOTCH1 protein was confirmed in four of the mutant cell lines tested (FIG. 3A). A missense mutation (C478F) that occurred in two cell lines derived from the same patient (HN31 and HN30) was confirmed to result in loss of NOTCH1 signaling upon ligand activation (Fig. 3B). Using an antibody that recognized only the activated form of NOTCH1 (cl-NOTCH1), no signal was present in mutant UM47 (G192*) or HN31 (C478F) (FIG. 3B) when cells were cultured on immobilized NOTCH1 ligand Jagged1 (Jag1) after infection with an empty control retrovirus (MigR1); however, infection with retrovirus restoring wild type (wt) full-length *NOTCH1* (NFL1) resulted in cl-NOTCH1 that increased greatly in cells grown on Jag1 (FIG. 3B). The presence of activated NOTCH1 are assessed by determination of the levels of expression of cleaved NOTCH1 (cl-NOTCH1) in the nucleus of tumor cells by immunohistochemistry (IHC) according to published methods (Kluk, Ashworth et al. 2013, Rettig, Chung et al. 2015) with an antibody that specifically detects cl-NOTCH (NICD; Notch intracellular domain) which is formed following activation of NOTCH1 at the plasma membrane (Nowell and Radtke 2017) (FIG. 3C).

### EXAMPLE 3

### NOTCH1-LOF mutants are more sensitive than NOTCH1wt cells to drugs targeting PI3K/mTOR.

Six different HNSCC cell lines with and without *NOTCH1* mutations were analyzed for their sensitivity towards bimiralisib. At 5 µM bimiralisib induces only cell death in HNSCC cell lines with *NOTCH1* mutations as determined by BrDU-TUNEL staining, while in HNSCC *NOTCH1wt* bimiralisib arrested cell lines in G1/S. Cell death as measured by BrdU-TUNEL was significantly increased in HNSCC cell lines with *NOTCH1* mutations but not in HNSCC lines with *NOTCH1wt* or *PIK3CA* mutations (FIG.4A). To further validate these findings an *in vivo* experiment was performed by sublingual implantation of 2 *NOTCH1* mutant HNSCC cell lines (UM22A and HN31) and one *NOTCH1*wt HNSCC cell line (FaDu) (FIG. 4B) followed by once daily PO treatment with bimiralisib (50 mg/kg) for 28 days. This regiment of bimiralisib was well tolerated during the whole treatment period of 28 days without body weight loss. There was significant reduction of tumor growth after treatment with bimiralisib drug in the 2 HNSCC tumors that harboured the *NOTCH1* inactivating mutations (*p<0.05) while the growth of the HNSCC tumor harboring the *NOTCHwt* was not affected by the bimiralisib treatment. These data clearly indicate that that cells harboring *NOTCH1* inactivating mutations are sensitive and vulnerable to the treatment of bimiralisib both *in vitro* and *in vivo.* In summary these data demonstrate that cell harboring *NOTCH1*-LoF mutation are sensitive both *in vitro* and *in vivo* to dual PI3K/mTOR inhibitor bimiralisib.

### EXAMPLE 4

### Treatment of a patient with metastatic head and neck squamous cell carcinoma harboring a NOTCH1 loss-of-function mutation

A human patient with heavily pretreated metastatic HNSCC with a SCC of the tongue harboring a *NOTCH1*-LoF mutation (L250fs*6) with a lung metastasis was treated with the very preferred PI3K/mTOR inhibitor bimiralisib. The L250fs*6 mutation is a frameshift mutation that occurs at amino acid 250 which will disrupt or lead to loss of function of *NOTCH1* (*6 indicates how many codons before reaching the new stop site). This mutation fully satisfies the criteria put forth in claim 1 c (i). Treatment with 140 mg of bimiralisib for 2 consecutive days followed by 5-day interval before the 140 mg treatment for two consecutive days was repeated or the whole treatment duration. The patient showed regression of a lung metastasis after 6 weeks of treatment with bimiralisib as determined by PET/CT; target lesions (metastases) of the patient had regressed remarkably (by more than 80%) (FIG. 5 - upper panel longitudinal scan and lower panel cross section). The patient remained on the study for 8 months until she passed away due to an event unrelated to bimiralisib. The maximal response was an 89% reduction in tumor size.

### EXAMPLE 5

### Treatment of patients with recurrent or metastatic HNSCCs containing NOTCH1 loss-of-function mutations

In the clinical study described thereafter, the very preferred PI3K/mTOR inhibitor of the present invention, bimiralisib, is administered orally in patients whose HNSCC harbor *NOTCH1*-LoF mutations, wherein said oral administration is effected as capsules comprising 20 mg and 80 mg of said bimiralisib. Further in detail, patients with recurrent or metastatic HNSCC without curative treatment options and harboring *NOTCH1* -LoF mutations will receive bimiralisib orally, twice a week. Thus, bimiralisib will be given once daily for two consecutive days followed by five days without treatment. The treatment is effected as long as the patient benefits and agrees to participate in the study. The study will end when all patients have been treated for at least six (6) months or have discontinued study participation for any reason, whichever comes first.

The objective response rate (ORR) according to the response evaluation criteria in solid tumors (RECIST, version 1.1; E.A. Eisenhauer et al; European Journal of Cancer 45 (2009) 228-247) is determined. Furthermore, Time to response (TTR), duration of response (DOR), time to treatment failure (TTF) and progression-free survival (PFS) as well as the bimiralisib plasma concentration are determined. In addition, quantitative and qualitative changes in circulating tumor DNA upon treatment with bimiralisib are determined.

Further in detail, at baseline (within 4 weeks prior to first dose of bimiralisib), prior to starting week 7 (± 7 days), i.e. 6 weeks after the first dose of bimiralisib, and every 6 weeks thereafter tumor measurements will be performed. ORR, TTR, DOR, TTF and PFS will be evaluated according to the response evaluation criteria in solid tumors (RECIST, version 1.1). Moreover, blood samples for circulating tumor DNA (ctDNA) analyses will be collected at baseline (day -7 to 0), at study Day 43 and at the end of treatment.

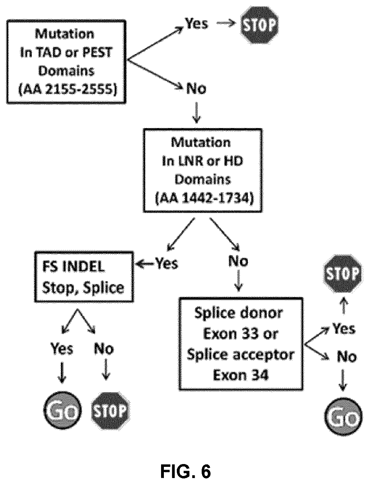

The inclusion criteria for this study are, in particular, as follows:
1. Male or female older than 18 years old.
2. Histologically or cytologically confirmed diagnosis of HNSCC, for which no standard curative or life prolonging therapy is available.
3. The tumor must harbor a *NOTCH1*-LoF mutation as confirmed by central review based on CLIA-certified NG sequencing results and in accordance with the present invention, and in particular with a preferred embodiment of the present invention as shown in FIG. 6. The latter implies that patients with activation *NOTCH1* mutations are excluded. Thus, patients with *NOTCH1* mutations that are predicted to be LoF by said preferred embodiment of the present invention will be eligible for enrolment. Essentially, we will exclude any mutations in the TAD or PEST domains, any missense or in-frame mutations in the LNR and HD domains, and splice donor (Exon 33) or acceptor (Exon 34) boundaries which would hypothetically truncate the protein within the TAD/PEST domains. All other *NOTCH1* mutations are eligible.
4. Measurable disease according to RECIST v.1.1 The exclusion criteria for this study are, in particular, as follows:
   1. Patient has an oncogenic K-ras mutation.
   2. Any anti-cancer treatment including investigational agents < 3 weeks, or palliative radiation < 2 weeks prior to the first dose of bimiralisib.

### Methods:

### (i) Analyzing NG sequencing data from pretreatment tumor biopsies of patient:

To determine eligibility for the study, available NG sequencing data from pretreatment tumor biopsies will be analyzed. NG sequencing data are generated by a CLIA-certified NG sequencing platform which covers the entire coding region of *NOTCH1.* Tumors will be measured every 6 weeks and evaluated using Response Evaluation Criteria in Solid Tumors (RECIST, version 1.1; E.A. Eisenhauer et al; European Journal of Cancer 45 (2009) 228-247).

### (ii) Determination of cleaved NOTCH1 (cl-NOTCH1; SEQ ID NO:3) in tumor material including tumor cells:

As indicated, cleaved NOTCH1 is a measure of pathway activation because ligand binding to the extracellular EGF-like repeats on NOTCH receptors creates mechanical tension exposing the molecule to stepwise cleavage at the S2 site by α-secretases and finally at the S3 cleavage site by γ-secretase to release intracellular cl-NOTCH1 which translocates to the nucleus and binds other transcription co-factors, altering expression of genes (Nowell and Radtke 2017).

The presence and residual levels of activated *NOTCH1* in tumors will be assessed by determination of the expression of cleaved NOTCH1 (cl-NOTCH1; SEQ ID NO:3) in tumor material including tumor cells, typically and preferably, by immunohistochemistry (IHC) according to published methods (Kluk, Ashworth et al. 2013, Rettig, Chung et al. 2015), with an antibody that specifically detects cl-NOTCH1. Paraffin-embedded pellets of wt and *NOTCH1* mutant HNSCC cell lines will be used as positive and negative controls. If IHC staining reveals nuclear cl-NOTCH1 staining in >10%, typically and preferably >5% of tumor material including tumor cells, then, in accordance with a preferred embodiment of the present invention, said patient may be replaced for the study as described in this Example.

### First results

A patient with pretreated metastatic HNSCC harboring a *NOTCH1*-LoF mutation (Q1037*) with lung metastases is being treated with the very preferred PI3K/mTOR inhibitor bimiralisib in the described study. No standard curative or life prolonging therapy was available for the patient. The Q1037* mutation introduces a stop codon at amino acid 1037, leading to loss of function of *NOTCH1.* This mutation fully satisfies the criteria put forth in claim 1 c (i). The patient has now been on bimiralisib treatment for over five (5) months and continues to benefit from treatment. Bimiralisib has completely stopped the growth of his metastases as evidenced by three (3) consecutive radiological tumor assessments (performed as per study protocol) after weeks 6, 12 and 18.

### REFERENCES

Agrawal, N., M. J. Frederick, C. R. Pickering, C. Bettegowda, K. Chang, R. J. Li, C. Fakhry, T. X. Xie, J. Zhang, J. Wang, N. Zhang, A. K. El-Naggar, S. A. Jasser, J. N. Weinstein, L. Trevino, J. A. Drummond, D. M. Muzny, Y. Wu, L. D. Wood, R. H. Hruban, W. H. Westra, W. M. Koch, J. A. Califano, R. A. Gibbs, D. Sidransky, B. Vogelstein, V. E. Velculescu, N. Papadopoulos, D. A. Wheeler, K. W. Kinzler and J. N. Myers (2011). "Exome sequencing of head and neck squamous cell carcinoma reveals inactivating mutations in NOTCH1." Science 333(6046): 1154-1157.
Agrawal, N., Y. Jiao, C. Bettegowda, S. M. Hutfless, Y. Wang, S. David, Y. Cheng, W. S. Twaddell, N. L. Latt, E. J. Shin, L.-D. Wang, L. Wang, W. Yang, V. E. Velculescu, B. Vogelstein, N. Papadopoulos, K. W. Kinzler and S. J. Meltzer (2012). "Comparative genomic analysis of esophageal adenocarcinoma and squamous cell carcinoma." Cancer discovery 2(10): 899-905.
Akbani, R., P. K. Ng, H. M. Werner, M. Shahmoradgoli, F. Zhang, Z. Ju, W. Liu, J. Y. Yang, K. Yoshihara, J. Li, S. Ling, E. G. Seviour, P. T. Ram, J. D. Minna, L. Diao, P. Tong, J. V. Heymach, S. M. Hill, F. Dondelinger, N. Stadler, L. A. Byers, F. Meric-Bernstam, J. N. Weinstein, B. M. Broom, R. G. Verhaak, H. Liang, S. Mukherjee, Y. Lu and G. B. Mills (2014). "A pan-cancer proteomic perspective on The Cancer Genome Atlas." Nat Commun 5: 3887.
Beaufils, F., N. Cmiljanovic, V. Cmiljanovic, T. Bohnacker, A. Melone, R. Marone, E. Jackson, X. Zhang, A. Sele, C. Borsari, J. Mestan, P. Hebeisen, P. Hillmann, B. Giese, M. Zvelebil, D. Fabbro, R. L. Williams, D. Rageot and M. P. Wymann (2017). "5-(4,6-Dimorpholino-1,3,5-triazin-2-yl)-4-(trifluoromethyl)pyridin-2-amine (PQR309), a Potent, Brain-Penetrant, Orally Bioavailable, Pan-Class I PI3K/mTOR Inhibitor as Clinical Candidate in Oncology." Journal of medicinal chemistry 60(17): 7524-7538.
Bohnacker, T., A. E. Prota, F. Beaufils, J. E. Burke, A. Melone, A. J. Inglis, D. Rageot, A. M. Sele, V. Cmiljanovic, N. Cmiljanovic, K. Bargsten, A. Aher, A. Akhmanova, J. F. Diaz, D. Fabbro, M. Zvelebil, R. L. Williams, M. O. Steinmetz and M. P. Wymann (2017). "Deconvolution of Buparlisib's mechanism of action defines specific PI3K and tubulin inhibitors for therapeutic intervention." Nat Commun 8: 14683.
Bornkamp, B., F. Bretz, H. Dette and J. Pinheiro (2011). "Response-adaptive dose-finding under model uncertainty." Ann. Appl. Stat. 5(2B): 1611-1631.
Byers, L. A., L. Diao, J. Wang, P. Saintigny, L. Girard, M. Peyton, L. Shen, Y. Fan, U. Giri, P. K. Tumula, M. B. Nilsson, J. Gudikote, H. Tran, R. J. Cardnell, D. J. Bearss, S. L. Warner, J. M. Foulks, S. B. Kanner, V. Gandhi, N. Krett, S. T. Rosen, E. S. Kim, R. S. Herbst, G. R. Blumenschein, J. J. Lee, S. M. Lippman, K. K. Ang, G. B. Mills, W. K. Hong, J. N. Weinstein, Wistuba, II, K. R. Coombes, J. D. Minna and J. V. Heymach (2013). "An Epithelial-Mesenchymal Transition Gene Signature Predicts Resistance to EGFR and PI3K Inhibitors and Identifies Axl as a Therapeutic Target for Overcoming EGFR Inhibitor Resistance." Clin Cancer Res 19(1): 279-290.
Byers, L. A., J. Wang, M. B. Nilsson, J. Fujimoto, P. Saintigny, J. Yordy, U. Giri, M. Peyton, Y. H. Fan, L. Diao, F. Masrorpour, L. Shen, W. Liu, B. Duchemann, P. Tumula, V. Bhardwaj, J. Welsh, S. Weber, B. S. Glisson, N. Kalhor, Wistuba, II, L. Girard, S. M. Lippman, G. B. Mills, K. R. Coombes, J. N. Weinstein, J. D. Minna and J. V. Heymach (2012). "Proteomic profiling identifies dysregulated pathways in small cell lung cancer and novel therapeutic targets including PARP1." Cancer Discov 2(9): 798-811.
Cancer Genome Atlas, N. (2015). "Comprehensive genomic characterization of head and neck squamous cell carcinomas." Nature 517(7536): 576-582.
Cerami, E., J. Gao, U. Dogrusoz, B. E. Gross, S. O. Sumer, B. A. Aksoy, A. Jacobsen, C. J. Byrne, M. L. Heuer, E. Larsson, Y. Antipin, B. Reva, A. P. Goldberg, C. Sander and N. Schultz (2012). "The cBio Cancer Genomics Portal: An Open Platform for Exploring Multidimensional Cancer Genomics Data." Cancer discovery 2(5): 401-404.
Courtney, K. D., R. B. Corcoran and J. A. Engelman (2010). "The PI3K pathway as drug target in human cancer." J Clin Oncol 28(6): 1075-1083.
D'Souza, B., A. Miyamoto and G. Weinmaster (2008). "The many facets of Notch ligands." Oncogene 27(38): 5148-5167.
Di Nicolantonio, F., S. Arena, J. Tabernero, S. Grosso, F. Molinari, T. Macarulla, M. Russo, C. Cancelliere, D. Zecchin, L. Mazzucchelli, T. Sasazuki, S. Shirasawa, M. Geuna, M. Frattini, J. Baselga, M. Gallicchio, S. Biffo and A. Bardelli (2010). "Deregulation of the PI3K and KRAS signaling pathways in human cancer cells determines their response to everolimus." The Journal of Clinical Investigation 120(8): 2858-2866.
Ferlay, J., I. Soerjomataram, R. Dikshit, S. Eser, C. Mathers, M. Rebelo, D. M. Parkin, D. Forman and F. Bray (2015). "Cancer incidence and mortality worldwide: Sources, methods and major patterns in GLOBOCAN 2012." International Journal of Cancer 136(5): E359-E386.
Ferrando, A. A. (2009). "The role of NOTCH1 signaling in T-ALL." Hematology / the Education Program of the American Society of Hematology. American Society of Hematology. Education Program: 353-361.
Ferrarotto, R., Y. Mitani, L. Diao, I. Guijarro, J. Wang, P. Zweidler-McKay, D. Bell, W. N. William, B. S. Glisson, M. J. Wick, A. M. Kapoun, A. Patnaik, G. Eckhardt, P. Munster, L. Farao, J. Dupont, J. J. Lee, A. Futreal, A. K. El-Naggar and J. V. Heymach (2017). "Activating NOTCH1 mutations define a distinct subgroup of adenoid cystic carcinoma patients with poor prognosis, propensity to bone and liver metastasis, and potential responsiveness to Notch1 inhibitors." Journal of clinical oncology : official journal of the American Society of Clinical Oncology 35(3): 352-360.
Fruman, D. A., H. Chiu, B. D. Hopkins, S. Bagrodia, L. C. Cantley and R. T. Abraham (2017). "The PI3K Pathway in Human Disease." Cell 170(4): 605-635.
Fruman, D. A. and C. Rommel (2014). "PI3K and cancer: lessons, challenges and opportunities." Nat Rev Drug Discov 13(2): 140-156.
Fukusumi, T and Califano J.A. (2018). "The NOTCH Pathway in Head and Neck Squamous Cell Carcinoma" Journal of Dental Research 97(6) 645-653.
Hales E. C, Taub J.W. Matherly LH. (2014) "New insights into Notch1 regulation of the PI3K-AKT-mTOR1 signaling axis: targeted therapy of gamma-secretase inhibitor resistant T-cell acute lymphoblastic leukemia". Cell Signal 26:149-61.
Ho, A. S., K. Kannan, D. M. Roy, L. G. T. Morris, I. Ganly, N. Katabi, D. Ramaswami, L. A. Walsh, S. Eng, J. T. Huse, J. Zhang, I. Dolgalev, K. Huberman, A. Heguy, A. Viale, M. Drobnjak, M. A. Leversha, C. E. Rice, B. Singh, N. G. Iyer, C. R. Leemans, E. Bloemena, R. L. Ferris, R. R. Seethala, B. E. Gross, Y. Liang, R. Sinha, L. Peng, B. J. Raphael, S. Turcan, Y. Gong, N. Schultz, S. Kim, S. Chiosea, J. P. Shah, C. Sander, W. Lee and T. A. Chan (2013). "The Mutational Landscape of Adenoid Cystic Carcinoma." Nature genetics 45(7): 791-798.
Iglesias-Bartolome, R., D. Martin and J. S. Gutkind (2013). "Exploiting the Head and Neck Cancer Oncogenome: Widespread PI3K-mTOR Pathway Alterations and Novel Molecular Targets." Cancer Discov 3(7): 722-725.
Janku, F., A. M. Tsimberidou, I. Garrido-Laguna, X. Wang, R. Luthra, D. S. Hong, A. Naing, G. S. Falchook, J. W. Moroney, S. A. Piha-Paul, J. J. Wheler, S. L. Moulder, S. Fu and R. Kurzrock (2011). "PIK3CA Mutations in Patients with Advanced Cancers Treated with PI3K/AKT/mTOR Axis Inhibitors." Molecular cancer therapeutics 10(3): 558-565.
Janku, F., J. J. Wheler, S. N. Westin, S. L. Moulder, A. Naing, A. M. Tsimberidou, S. Fu, G. S. Falchook, D. S. Hong, I. Garrido-Laguna, R. Luthra, J. J. Lee, K. H. Lu and R. Kurzrock (2012). "PI3K/AKT/mTOR inhibitors in patients with breast and gynecologic malignancies harboring PIK3CA mutations." J Clin Oncol 30(8): 777-782.
Jimeno, A., J. E. Bauman, C. Weissman, D. Adkins, I. Schnadig, P. Beauregard, D. W. Bowles, A. Spira, B. Levy, N. Seetharamu, D. Hausman, L. Walker, C. M. Rudin and K. Shirai (2015). "A randomized, phase 2 trial of docetaxel with or without PX-866, an irreversible oral phosphatidylinositol 3-kinase inhibitor, in patients with relapsed or metastatic head and neck squamous cell cancer." Oral Oncol 51(4): 383-388.
Johnson, F. M., V. Sambandam, L. Shen, M. Zhang, R. Saigal, P. Tong, T. Mazumdar, L. A. Byers, C. Pickering, J. N. Myers, J. Wang and M. Frederick (2016). "Abstract 393: NOTCH1 inactivating mutation mediates sensitivity to PI3K/mTOR inhibitors in head and neck squamous cell carcinoma." Cancer Research 76(14 Supplement): 393-393.
Kluk, M. J., T. Ashworth, H. Wang, B. Knoechel, E. F. Mason, E. A. Morgan, D. Dorfman, G. Pinkus, O. Weigert, J. L. Hornick, L. R. Chirieac, M. Hirsch, D. J. Oh, A. P. South, I. M. Leigh, C. Pourreyron, A. J. Cassidy, D. J. DeAngelo, D. M. Weinstock, I. E. Krop, D. Dillon, J. E. Brock, A. J. F. Lazar, M. Peto, R. J. Cho, A. Stoeck, B. B. Haines, S. Sathayanrayanan, S. Rodig and J. C. Aster (2013). "Gauging NOTCH1 Activation in Cancer Using Immunohistochemistry." PLOS ONE 8(6): e67306.
Lui, V. W., M. L. Hedberg, H. Li, B. S. Vangara, K. Pendleton, Y. Zeng, Y. Lu, Q. Zhang, Y. Du, B. R. Gilbert, M. Freilino, S. Sauerwein, N. D. Peyser, D. Xiao, B. Diergaarde, L. Wang, S. Chiosea, R. Seethala, J. T. Johnson, S. Kim, U. Duvvuri, R. L. Ferris, M. Romkes, T. Nukui, P. Kwok-Shing Ng, L. A. Garraway, P. S. Hammerman, G. B. Mills and J. R. Grandis (2013). "Frequent mutation of the PI3K pathway in head and neck cancer defines predictive biomarkers." Cancer Discov 3(7): 761-769.
Maira, S. M. (2011). "PI3K inhibitors for cancer treatment: five years of preclinical and clinical research after BEZ235." Mol Cancer Ther 10(11): 2016.
Mao, L. (2015). "NOTCH Mutations: Multiple Faces in Human Malignancies." Cancer Prevention Research 8(4): 259-261.
Maxwell, P., S. O. Hynes, M. Fuchs, S. Craig, C. McGready, F. McLean, S. McQuaid, J. James and M. Salto-Tellez (2018). "Practical guide for the comparison of two next-generation sequencing systems for solid tumour analysis in a universal healthcare system." Journal of Clinical Pathology.
Mazumdar, T., L. A. Byers, P. K. Ng, G. B. Mills, S. Peng, L. Diao, Y. H. Fan, K. Stemke-Hale, J. V. Heymach, J. N. Myers, B. S. Glisson and F. M. Johnson (2014). "A Comprehensive Evaluation of Biomarkers Predictive of Response to PI3K Inhibitors and of Resistance Mechanisms in Head and Neck Squamous Cell Carcinoma." Mol Cancer Ther 13(11): 2738-2750.
Nowell, C. S. and F. Radtke (2017). "Notch as a tumour suppressor." Nat Rev Cancer 17(3): 145-159.
Pickering, C. R., J. Zhang, S. Y. Yoo, L. Bengtsson, S. Moorthy, D. M. Neskey, M. Zhao, M. V. Ortega Alves, K. Chang, J. Drummond, E. Cortez, T. X. Xie, D. Zhang, W. Chung, J. P. Issa, P. A. Zweidler-McKay, X. Wu, A. K. El-Naggar, J. N. Weinstein, J. Wang, D. M. Muzny, R. A. Gibbs, D. A. Wheeler, J. N. Myers and M. J. Frederick (2013). "Integrative genomic characterization of oral squamous cell carcinoma identifies frequent somatic drivers." Cancer Discov 3(7): 770-781.
Pickering, C. R., J. H. Zhou, J. J. Lee, J. A. Drummond, S. A. Peng, R. E. Saade, K. Y. Tsai, J. L. Curry, M. T. Tetzlaff, S. Y. Lai, J. Yu, D. M. Muzny, H. Doddapaneni, E. Shinbrot, K. R. Covington, J. Zhang, S. Seth, C. Caulin, G. L. Clayman, A. K. El-Naggar, R. A. Gibbs, R. S. Weber, J. N. Myers, D. A. Wheeler and M. J. Frederick (2014). "Mutational landscape of aggressive cutaneous squamous cell carcinoma." Clinical cancer research : an official journal of the American Association for Cancer Research 20(24): 6582-6592.
Pounds, S. and S. W. Morris (2003). "Estimating the occurrence of false positives and false negatives in microarray studies by approximating and partitioning the empirical distribution of p-values." Bioinformatics 19(10): 1236-1242.
Rettig, E. M., C. H. Chung, J. A. Bishop, J. D. Howard, R. Sharma, R. J. Li, C. Douville, R. Karchin, E. Izumchenko, D. Sidransky, W. Koch, J. Califano, N. Agrawal and C. Fakhry (2015). "Cleaved NOTCH1 expression pattern in head and neck squamous cell carcinoma is associated with NOTCH1 mutation, HPV status and high-risk features." Cancer prevention research (Philadelphia, Pa.) 8(4): 287-295.
Ritz, C. and J. C. Streibig (2005). "Bioassay Analysis Using R." Journal of Statistical Software; Vol 1, Issue 5 (2005).
Rodon, J., R. Dienstmann, V. Serra and J. Tabernero (2013). "Development of PI3K inhibitors: lessons learned from early clinical trials." Nat Rev Clin Oncol 10(3): 143-153.
Sambandam, V., L. Shen, P. Tong, T. Mazumdar, C. Pickering, J. Myers, J. Wang, M. Frederick and F. Johnson (2017). Abstract 2992: Identification of NOTCH1 inactivating mutation as a therapeutic vulnerability to PI3K/mTOR pathway inhibition in head and neck squamous cell carcinoma (HNSCC).
Sambandam, V., L. Shen, P. Tong, T. Mazumdar, C. Pickering, J. N. Myers, J. Wang, M. Frederick and F. M. Johnson (2017). "Abstract 2992: Identification of <em>NOTCH1</em> inactivating mutation as a therapeutic vulnerability to PI3K/mTOR pathway inhibition in head and neck squamous cell carcinoma (HNSCC)." Cancer Research 77(13 Supplement): 2992-2992.
Stransky, N., A. M. Egloff, A. D. Tward, A. D. Kostic, K. Cibulskis, A. Sivachenko, G. V. Kryukov, M. S. Lawrence, C. Sougnez, A. McKenna, E. Shefler, A. H. Ramos, P. Stojanov, S. L. Carter, D. Voet, M. L. Cortes, D. Auclair, M. F. Berger, G. Saksena, C. Guiducci, R. C. Onofrio, M. Parkin, M. Romkes, J. L. Weissfeld, R. R. Seethala, L. Wang, C. Rangel-Escareno, J. C. Fernandez-Lopez, A. Hidalgo-Miranda, J. Melendez-Zajgla, W. Winckler, K. Ardlie, S. B. Gabriel, M. Meyerson, E. S. Lander, G. Getz, T. R. Golub, L. A. Garraway and J. R. Grandis (2011). "The mutational landscape of head and neck squamous cell carcinoma." Science 333(6046): 1157-1160.
The Cancer Genome Atlas, N. (2015). "Comprehensive genomic characterization of head and neck squamous cell carcinomas." Nature 517(7536): 576-582.
Wicki, A., N. Brown, A. Xyrafas, V. Bize, H. Hawle, S. Berardi, N. Cmiljanovi, V.ć Cmiljanović, M. Stumm, S. Dimitrijević, R. Herrmann, V. Prêtre, R. Ritschard, A. Tzankov, V. Hess, A. Childs, C. Hierro, J. Rodon, D. Hess, M. Joerger, R. von Moos, C. Sessa and R. Kristeleit (2018). "First-in human, phase 1, dose-escalation pharmacokinetic and pharmacodynamic study of the oral dual PI3K and mTORC1/2 inhibitor PQR309 in patients with advanced solid tumors (SAKK 67/13)." European Journal of Cancer 96: 6-16.

## Claims

1. A pharmaceutical composition comprising a therapeutically effective amount of bimiralisib, for use in treatment of a squamous cell carcinoma (SCC) of a human patient, wherein
said human patient's SCC harbors one or more *NOTCH1* mutations, wherein said *NOTCH1* mutation is a *NOTCH1* loss of function mutation.

2. The pharmaceutical composition for use of claim 1, wherein
(i) the *NOTCH1* loss of function mutation is not
a. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2;
b. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; or
c. a mutation in the splice donor boundary (Exon 33), or in the acceptor boundary (Exon 34) of said *NOTCH1* gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1; or
(ii) the human patient's SCC comprises cleaved NOTCH1 intracellular domain protein (cl-NOTCH1, [NICD1]; SEQ ID NO:3) in an amount incompatible with NOTCH1 loss-of-function; or
(iii) a combination of (i) and (ii).

3. The pharmaceutical composition for use of claim 1, wherein
(i) the *NOTCH1* loss of function mutation is not
a. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2;
b. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2; or
c. a mutation in the splice donor boundary (Exon 33), or in the acceptor boundary (Exon 34) of said *NOTCH1* gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1.

4. The pharmaceutical composition for use of claim 3, wherein the mutation in the NOTCH1 gene is not in the PEST domain of said human *NOTCH1* gene corresponding to aa 2159-2555 of SEQ ID NO:2.

5. The pharmaceutical composition for use of claim 3, wherein the mutation in the NOTCH1 gene is not in the heterodimerization domain (HD domain) of said human *NOTCH1* gene corresponding to aa 1442-1734 of SEQ ID NO:2.

6. The pharmaceutical composition for use of claim 3, wherein the mutation in the NOTCH1 gene is not within nucleotides 5639-6082 of SEQ ID NO:1.

7. The pharmaceutical composition for use of claim 1, wherein the SCC has any one or more of the following mutations: p.Q1957*, p.C478F, p.G192*, Q1037*, and L250fs*; preferably p.C478F, p.G192*, or L250fs*.

8. The pharmaceutical composition for use of claim 1 or 2, wherein the human patient's SCC comprises cleaved NOTCH1 intracellular domain protein (cl-NOTCH1, [NICD1]; SEQ ID NO:3) in an amount incompatible with NOTCH1 loss-of-function.

9. The pharmaceutical composition for use of claim 8, wherein the cleaved NOTCH1 intracellular domain protein (cl-NOTCH1, [NICD1]; SEQ ID NO:3) is cleaved NOTCH1 intracellular domain protein in the nuclei of the SCC cells.

10. The pharmaceutical composition for use of any of the preceding claims, wherein the squamous cell carcinoma is a head and neck squamous cell carcinoma (HNSCC), a skin squamous cell carcinoma, an esophagus squamous cell carcinoma, or a lung squamous cell carcinoma; preferably wherein the squamous cell carcinoma is a head and neck squamous cell carcinoma (HNSCC).

11. The pharmaceutical composition for use of any of the preceding claims, wherein the presence of the *NOTCH1* mutation is established by identifying the status of a biomarker from tumor material from said human patient.

12. The pharmaceutical composition for use of claim 11, wherein the biomarker is selected from the group consisting of
a. sequenced tumor DNA, preferably sequenced human tumor DNA, to identify one or more mutations in the NOTCH1 gene, preferably in the human *NOTCH1* gene of SEQ ID NO:1, encoding the NOTCH1 protein, preferably the human NOTCH1 protein of SEQ ID NO:2;
b. protein level of cleaved NOTCH1 intracellular domain (cl-NOTCH1, [NICD1]; SEQ ID NO:3), wherein preferably said NICD1 is determined by immunohistochemistry (IHC); and
c. a combination of biomarker (i) and (ii).

13. The pharmaceutical composition for use of claim 12, wherein said identifying the status of a biomarker from tumor material from said human patient further comprises comparing the status of the biomarker in said tumor material to a normal status of the biomarker.

14. The pharmaceutical composition for use of claim 13, said normal status of said biomarker is the status of said biomarker in a non-cancerous, healthy, or wild-type tissue or cell.

15. The pharmaceutical composition for use of claim 13 or 14, wherein said identifying the status of a biomarker from tumor material from said human patient further comprises selecting the human patient as being predicted to benefit from said treatment with bimiralisib, if
a. said human patient's SCC harbors one or more *NOTCH1* mutations, wherein said *NOTCH1* mutation is not
i. a mutation in the TAD domain or in the PEST domain of said *NOTCH1* gene, and wherein preferably said *NOTCH1* mutation is not a mutation in the TAD domain or in the PEST domain of said human *NOTCH1* gene corresponding to aa 2159-2555 of SEQ ID NO:2;
ii. a missense or an in-frame mutation, preferably a missense or an in-frame mutation incompatible with NOTCH1 loss-of-function, in the Lin-12/Notch 1 Repeats (LNR) or in the heterodimerization domain (HD domain) of said *NOTCH1* gene, and wherein preferably said *NOTCH1* mutation is not a missense or not an in-frame mutation, further preferably not a missense or an in-frame mutation incompatible with NOTCH1 loss-of-function, in the Lin-12/Notch 1 Repeats (LNR) or in the heterodimerization domain (HD domain) of said human *NOTCH1* gene corresponding to aa 1442-1734 of SEQ ID NO:2 (full length human NOTCH1 protein]
iii. a mutation in the splice donor boundary (Exon 33), or in the acceptor boundary (Exon 34) of said NOTCH1 gene, and wherein preferably said *NOTCH1* mutation is not a mutation in the splice donor boundary (Exon 33), or in the acceptor boundary (Exon 34) of said human *NOTCH1* gene corresponding to nt 5639-6082 of SEQ ID NO:1; or
b. said human patient's SCC comprises cleaved NOTCH1 intracellular domain protein (cl-NOTCH1, [NICD1]; SEQ ID NO:3) in an amount incompatible with NOTCH1 loss-of-function; or
c. a combination of a. and b.
